# EUROPEAN PATENT APPLICATION

(11) **EP 4 726 046 A1**
(43) Date of publication of application: **15.04.2026**
(21) Application number: 24823406.4
(22) Date of filing: 12.06.2024
(51) Int. Cl.: C12P 7/22, C08G 8/10, C08G 63/60, C12N 1/21, C12P 7/42

(54) **AROMATIC COMPOUND COMPOSITION AND POLYMER**

(30) Priority: 12.06.2023 JP 2023096317; 12.06.2023 JP 2023096338; 12.06.2023 JP 2023096350
(71) Applicant: Green Chemicals Co., Ltd., Kizugawa-shi Kyoto 619-0292 (JP)
(72) Inventor: MURATA Ryuichi, Fujieda-shi, Shizuoka 426-0041 (JP); INOUE Yusuke, Fujieda-shi, Shizuoka 426-0041 (JP); TACHIBANA Kenya, Fujieda-shi, Shizuoka 426-0041 (JP); FUJIWARA Daisuke, Fujieda-shi, Shizuoka 426-0041 (JP); HASHIZUME Masayoshi, Fujieda-shi, Shizuoka 426-0041 (JP); INUI Masayuki, Kizugawa-shi, Kyoto 619-0292 (JP); HIRAGA Kazumi, Kizugawa-shi, Kyoto 619-0292 (JP); KITADE Yukihiro, Kizugawa-shi, Kyoto 619-0292 (JP); SUDA Masako, Kizugawa-shi, Kyoto 619-0292 (JP); HASHIMOTO Ryoma, Kizugawa-shi, Kyoto 619-0292 (JP)
(74) Representative: Ter Meer Steinmeister & Partner
(86) International application number: PCT/JP2024/021322
(87) International publication number: WO 2024/257791

(57) **Abstract**

An aromatic compound composition contains at least an aromatic compound (A) in which an actually measured biobased content representing a proportion of radioactive carbon ¹⁴C derived from a biomass resource, the actually measured biobased content being determined based on a concentration of radioactive carbon in circulating carbon in the 1950s as measured in accordance with ASTM D6866-20, is equal to or more than 80% and equal to or less than 100%, in which the aromatic compound (A) is phenol (A) or 4-hydroxybenzoic acid (A), in a case where the aromatic compound (A) is the phenol (A), an absorbance at a wavelength of 425 nm when the aromatic compound composition is dissolved in pure water to have a concentration of 90% by mass is equal to or more than 0.001 and equal to or less than 0.70, and in a case where the aromatic compound (A) is the 4-hydroxybenzoic acid (A), an absorbance at a wavelength of 425 nm when the aromatic compound composition is dissolved in ethanol at a concentration of 20 g/L is equal to or less than 0.015, or a content of shikimic acid in the aromatic compound composition is equal to or less than 0.012% by mass with respect to 100% by mass of the entire aromatic compound composition.

## Description

### TECHNICAL FIELD

The present invention relates to an aromatic compound composition and a polymer.

### BACKGROUND ART

In the background of global warming and the problem of depletion of fossil resources, manufacturing of chemicals using renewable biomass resources as a raw material has attracted attention as a new industry for realizing a low-carbon society. Examples of such chemicals include phenol derived from biomass.

As a technology related to the phenol derived from biomass, for example, phenol disclosed in Patent Document 1 is known. Patent Document 1 discloses a phenolic resin including a structure derived from specific plant-derived phenols, a structure derived from other phenols, a structure derived from aldehydes, and a structure derived from saccharides.

In addition, 4-hydroxybenzoic acid (4-HBA) is a useful chemical substance used as a raw material of a liquid crystal polymer, a synthesis raw material of paraben which is a preservative, and the like.

At present, the 4-hydroxybenzoic acid is chemically produced using crude oil as a raw material. A method for chemically manufacturing the 4-hydroxybenzoic acid includes, for example, a method of reacting phenol, potassium hydroxide, and carbon dioxide under high pressure conditions.

However, in such a method, not only is phenol used as a raw material dependent on a fossil raw material, but also a strong alkali, carbon dioxide, and high-temperature and high-pressure conditions are required in the reaction process, and harmful waste liquid and exhaust gas are generated in the manufacturing process, which imposes a large burden on the environment.

In view of such a background, it is strongly desired to establish a technology for manufacturing the 4-hydroxybenzoic acid by a biological method using a renewable resource as a raw material, which is energy-saving and reduces the harmful waste liquid.

For example, it is clarified that, using Escherichia coli, the 4-HBA is synthesized from chorismic acid, which is an intermediate in the shikimic acid pathway involved in the synthesis of aromatic amino acids and the like, by chorismate-pyruvate lyase encoded by ubiC, in Non-Patent Documents 1 and 2 and Patent Documents 2 and 3.

### RELATED DOCUMENT

### PATENT DOCUMENT

[Non-Patent Document 1] J. Bacteriol., 174, 5309-5316 (1992)
[Non-Patent Document 2] Microbiology, 140, 897-904 (1994)
[Patent Document 1] Japanese Unexamined Patent Publication No. 2017-119765
[Patent Document 2] United States Patent No. 6030819
[Patent Document 3] United States Patent No. 6114157

### SUMMARY OF THE INVENTION

### TECHNICAL PROBLEM

According to the studies of the present inventors, it has been clarified that an aromatic compound composition derived from biomass has room for improvement from the viewpoint of improving balance between environmental compatibility and color tone or polymerizability.

The present invention has been made in view of the above circumstances, and provides an aromatic compound composition in which the balance between environmental compatibility and color tone or polymerizability is improved.

In addition, according to the studies of the present inventors, it has been clarified that a phenol composition derived from biomass has room for improvement from the viewpoint of color tone of a polymer synthesized using the phenol composition.

A first embodiment of the present invention has been made in view of the above circumstances, and provides a phenol composition in which the balance between environmental compatibility and color tone of a polymer to be obtained is improved.

In addition, according to the studies of the present inventors, it has been clarified that, in a case where the 4-hydroxybenzoic acid derived from biomass as described above is used for polymerization of a resin which is a downstream product, there is room for improvement in polymerizability.

A second embodiment of the present invention has been made in view of the above circumstances, and provides a 4-hydroxybenzoic acid composition and a polymer, in which the balance between environmental compatibility and polymerizability is improved.

In addition, according to the studies of the present inventors, it has been clarified that the 4-hydroxybenzoic acid derived from biomass as described above has room for improvement from the viewpoint of color tone.

A third embodiment of the present invention has been made in view of the above circumstances, and provides a 4-hydroxybenzoic acid composition in which the balance between environmental compatibility and color tone is improved.

### SOLUTION TO PROBLEM

According to the present invention, there is provided an aromatic compound composition containing at least an aromatic compound (A) in which an actually measured biobased content representing a proportion of radioactive carbon ¹⁴C derived from a biomass resource, the actually measured biobased content being determined based on a concentration of radioactive carbon in circulating carbon in the 1950s as measured in accordance with ASTM D6866-20, is equal to or more than 80% and equal to or less than 100%, in which the aromatic compound (A) is phenol (A) or 4-hydroxybenzoic acid (A), in a case where the aromatic compound (A) is the phenol (A), an absorbance at a wavelength of 425 nm when the aromatic compound composition is dissolved in pure water to have a concentration of 90% by mass is equal to or more than 0.001 and equal to or less than 0.70, and in a case where the aromatic compound (A) is the 4-hydroxybenzoic acid (A), an absorbance at a wavelength of 425 nm when the aromatic compound composition is dissolved in ethanol at a concentration of 20 g/L is equal to or less than 0.015, or a content of shikimic acid in the aromatic compound composition is equal to or less than 0.012% by mass with respect to 100% by mass of the entire aromatic compound composition.

In addition, the present inventors have made intensive studies to achieve the above-described object. As a result, it has been found that the balance between environmental compatibility and color tone of a polymer to be obtained can be improved by setting the absorbance of light having a wavelength of 425 nm to a specific range, and thus the first embodiment of the present invention has been completed.
[1a] A phenol composition containing at least phenol (A) in which an actually measured biobased content representing a proportion of radioactive carbon ¹⁴C derived from a biomass resource, which is determined based on a concentration of radioactive carbon in circulating carbon in the 1950s as measured in accordance with ASTM D6866-20, is equal to or more than 80% and equal to or less than 100%, and an absorbance at a wavelength of 425 nm when the phenol composition is dissolved in pure water to have a concentration of 90% by mass is equal to or more than 0.001 and equal to or less than 0.70.
[2a] The phenol composition according to [1a], in which a content of the phenol (A) is equal to or more than 95% by mass and equal to or less than 100% by mass.
[3a] The phenol composition according to [1a] or [2a], in which the actually measured biobased content of the phenol (A) is equal to or more than 95% and equal to or less than 100%.
[4a] The phenol composition according to any one of [1a] to [3a], in which the absorbance is equal to or less than 0.62.
[5a] The phenol composition according to any one of [1a] to [4a], in which the phenol (A) includes at least a structure derived from a benzene ring of a product of one or two or more transformants selected from a transformant HBA-2 (Accession No.: NITE BP-01838) and a transformant HBA-47 (Accession No.: NITE BP-01849).
[6a] The phenol composition according to any one of [1a] to [5a], in which the phenol (A) includes at least a product of one or two or more transformants selected from a transformant PHE7 (Accession No.: NITE BP-976), a transformant PHE18 (Accession No.: NITE BP-995), a transformant PHE21 (Accession No.: NITE BP-996), and a transformant PHE31 (Accession No.: NITE BP-999).
[7a] A polymer including at least a structural unit derived from the phenol (A) in the phenol composition according to any one of [1a] to [6a].
[8a] The polymer according to [7a], in which the polymer is a phenolic resin.

In addition, the present inventors have made intensive studies to achieve the above-described object. As a result, it has been found that the balance between environmental compatibility and polymerizability is improved by setting the content of shikimic acid in the 4-hydroxybenzoic acid composition to be equal to or less than a predetermined concentration, and thus the second embodiment of the present invention has been completed.
[1b] A 4-hydroxybenzoic acid composition containing at least 4-hydroxybenzoic acid (A) in which an actually measured biobased content representing a proportion of radioactive carbon ¹⁴C derived from a biomass resource, which is determined based on a concentration of radioactive carbon in circulating carbon in the 1950s as measured in accordance with ASTM D6866-20, is equal to or more than 80% and equal to or less than 100%, and a content of shikimic acid is equal to or less than 0.012% by mass with respect to 100% by mass of the entire 4-hydroxybenzoic acid composition.
[2b] The 4-hydroxybenzoic acid composition according to [1b], in which a content of the 4-hydroxybenzoic acid (A) is equal to or more than 95% by mass and equal to or less than 100% by mass with respect to 100% by mass of the entire 4-hydroxybenzoic acid composition.
[3b] The 4-hydroxybenzoic acid composition according to [1b] or [2b], in which the actually measured biobased content of the 4-hydroxybenzoic acid (A) is equal to or more than 95% and equal to or less than 100%.
[4b] The 4-hydroxybenzoic acid composition according to any one of [1b] to [3b], in which the content of shikimic acid is equal to or less than 0.008% by mass with respect to 100% by mass of the entire 4-hydroxybenzoic acid composition.
[5b] The 4-hydroxybenzoic acid composition according to any one of [1b] to [4b], in which the 4-hydroxybenzoic acid (A) includes at least a product of one or two transformants selected from a transformant HBA-2 (Accession No.: NITE BP-01838) and a transformant HBA-47 (Accession No.: NITE BP-01849).
[6b] A polymer including at least a structure of the 4-hydroxybenzoic acid (A) derived from the 4-hydroxybenzoic acid composition according to any one of [1b] to [5b].
[7b] The polymer according to [6b], in which the polymer is a liquid crystal polymer.

In addition, the present inventors have made intensive studies to achieve the above-described object. As a result, it has been found that the balance between environmental compatibility and color tone of the 4-hydroxybenzoic acid composition can be improved by setting the absorbance of light having a wavelength of 425 nm to a specific range, and thus the third embodiment of the present invention has been completed.
[1c] A 4-hydroxybenzoic acid composition containing at least 4-hydroxybenzoic acid (A) in which an actually measured biobased content representing a proportion of radioactive carbon ¹⁴C derived from a biomass resource, which is determined based on a concentration of radioactive carbon in circulating carbon in the 1950s as measured in accordance with ASTM D6866-20, is equal to or more than 80% and equal to or less than 100%, and an absorbance at a wavelength of 425 nm when the 4-hydroxybenzoic acid composition is dissolved in ethanol at a concentration of 20 g/L is equal to or less than 0.015.
[2c] The 4-hydroxybenzoic acid composition according to [1c], in which a content of the 4-hydroxybenzoic acid (A) is equal to or more than 95% by mass and equal to or less than 100% by mass with respect to 100% by mass of the entire 4-hydroxybenzoic acid composition.
[3c] The 4-hydroxybenzoic acid composition according to [1c] or [2c], in which the actually measured biobased content of the 4-hydroxybenzoic acid (A) is equal to or more than 95% and equal to or less than 100%.
[4c] The 4-hydroxybenzoic acid composition according to any one of [1c] to [3c], in which the absorbance is equal to or less than 0.010.
[5c] The 4-hydroxybenzoic acid composition according to any one of [1c] to [4c], in which the absorbance is equal to or more than 0.001.
[6c] The 4-hydroxybenzoic acid composition according to any one of [1c] to [5c], in which the 4-hydroxybenzoic acid (A) includes at least a product of one or two transformants selected from a transformant HBA-2 (Accession No.: NITE BP-01838) and a transformant HBA-47 (Accession No.: NITE BP-01849).

### ADVANTAGEOUS EFFECTS OF INVENTION

According to the present invention, it is possible to provide an aromatic compound composition in which the balance between environmental compatibility and color tone or polymerizability is improved.

In addition, according to the first embodiment of the present invention, it is possible to provide a phenol composition in which the balance between environmental compatibility and color tone of a polymer to be obtained is improved.

In addition, according to the second embodiment of the present invention, it is possible to provide a 4-hydroxybenzoic acid composition and a polymer, in which the balance between environmental compatibility and polymerizability is improved.

In addition, according to the third embodiment of the present invention, it is possible to provide a 4-hydroxybenzoic acid composition in which the balance between environmental compatibility and color tone is improved.

### DESCRIPTION OF EMBODIMENTS

Hereinafter, embodiments of the present invention will be described in detail.

In the present embodiment, "pure water" refers to water having a conductivity of equal to or more than 1 MΩ·cm.

In the present specification, "liquid crystal polymer" generally refers to a polymer that can have a rod-like structure for exhibiting liquid crystalline behavior (for example, a thermotropic nematic state) in a molten state.

The aromatic compound composition according to the present embodiment contains at least an aromatic compound (A) in which an actually measured biobased content representing a proportion of radioactive carbon ¹⁴C derived from a biomass resource, the actually measured biobased content being determined based on a concentration of radioactive carbon in circulating carbon in the 1950s as measured in accordance with ASTM D6866-20, is equal to or more than 80% and equal to or less than 100%, in which the aromatic compound (A) is phenol (A) or 4-hydroxybenzoic acid (A), in a case where the aromatic compound (A) is the phenol (A), an absorbance at a wavelength of 425 nm when the aromatic compound composition is dissolved in pure water to have a concentration of 90% by mass is equal to or more than 0.001 and equal to or less than 0.70, and in a case where the aromatic compound (A) is the 4-hydroxybenzoic acid (A), an absorbance at a wavelength of 425 nm when the aromatic compound composition is dissolved in ethanol at a concentration of 20 g/L is equal to or less than 0.015, or a content of shikimic acid in the aromatic compound composition is equal to or less than 0.012% by mass with respect to 100% by mass of the entire aromatic compound composition.

Hereinafter, more specific aspects of the present embodiment will be described as a first embodiment to a third embodiment.

### <First embodiment>

### [Phenol composition]

The phenol composition according to the first embodiment contains at least phenol (A) in which an actually measured biobased content representing a proportion of radioactive carbon ¹⁴C derived from a biomass resource, which is determined based on a concentration of radioactive carbon in circulating carbon in the 1950s as measured in accordance with ASTM D6866-20, is equal to or more than 80% and equal to or less than 100%, and an absorbance at a wavelength of 425 nm when the phenol composition is dissolved in pure water to have a concentration of 90% by mass is equal to or more than 0.001 and equal to or less than 0.70.

In such a phenol composition, the actually measured biobased content indicates the amount of carbon derived from a plant in the phenol (A); and as the value is larger, the phenol composition is composed of a natural raw material. Therefore, as the actually measured biobased content is larger, environmental compatibility is favorable from the viewpoint of carbon neutrality. In addition, in a case where the above-described absorbance is equal to or more than 0.001 and equal to or less than 0.70, it is possible to obtain a phenol composition in which color tone of a polymer obtained using the phenol composition is more suitable even in a case where the raw material derived from a plant is used.

Such a phenol composition can be produced by a manufacturing method described later, particularly by (i) generating a precursor from a raw material compound derived from a plant, using a first transformant, (ii) generating a phenol composition from the precursor using a second transformant, and (iii) isolating and concentrating the generated phenol composition.

In the phenol composition according to the first embodiment, the actually measured biobased content representing the proportion of radioactive carbon ¹⁴C derived from a biomass resource, which is determined based on a concentration of radioactive carbon in circulating carbon in the 1950s as measured in accordance with ASTM D6866-20, is equal to or more than 80% and equal to or less than 100%, preferably equal to or more than 85% and equal to or less than 100%, more preferably equal to or more than 90% and equal to or less than 100%, and still more preferably equal to or more than 95% and equal to or less than 100%. In a case where the above-described actually measured biobased content is within the above-described numerical range, the environmental compatibility is further improved from the viewpoint of carbon neutrality.

In this case, the actually measured biobased content representing the proportion of radioactive carbon ¹⁴C in all carbon atoms in the phenol (A) of the phenol composition is calculated from a content of radioactive carbon ¹⁴C in a sample measured based on a biobased concentration test specification ASTM D6866-20 defined by American Society for Testing and Materials (ASTM), and a concentration of the radioactive carbon ¹⁴C in circulating carbon in the 1950s. A plurality of analysis methods are specified in the test specification, and among these, an analysis method B (AMS method) is preferably used. For example, an analysis device such as an accelerator mass spectrometer Pelletron AMS manufactured by NEC Corporation is used for the analysis method B.

In the phenol composition according to the first embodiment, the upper limit value of the absorbance at a wavelength of 425 nm when the phenol composition is dissolved in pure water to have a concentration of 90% by mass is equal to or less than 0.70, preferably equal to or less than 0.65, more preferably equal to or less than 0.62, still more preferably equal to or less than 0.60, even more preferably equal to or less than 0.50, even still more preferably equal to or less than 0.40, further more preferably equal to or less than 0.25, even further more preferably equal to or less than 0.20, particularly preferably equal to or less than 0.15, and more particularly preferably equal to or less than 0.10. In a case where the absorbance at a wavelength of 425 nm is equal to or less than the above-described upper limit value, the color tone of the polymer obtained using the phenol composition according to the first embodiment is more suitable.

In addition, the lower limit value of the absorbance at a wavelength of 425 nm is not particularly limited, but is, for example, equal to or more than 0.001, and from the viewpoint of manufacturing efficiency, it is preferably equal to or more than 0.005, more preferably equal to or more than 0.01, still more preferably equal to or more than 0.02, even more preferably equal to or more than 0.03, even still more preferably equal to or more than 0.05, and further more preferably equal to or more than 0.07.

In the phenol composition according to the first embodiment, a content of the above-described phenol (A) is preferably equal to or more than 95% by mass and equal to or less than 100% by mass, more preferably equal to or more than 96% by mass and equal to or less than 100% by mass, still more preferably equal to or more than 97% by mass and equal to or less than 100% by mass, even more preferably equal to or more than 98% by mass and equal to or less than 100% by mass, and even still more preferably equal to or more than 99% by mass and equal to or less than 100% by mass, with respect to 100% by mass of the entire phenol composition. In a case where the content of the above-described phenol (A) is within the above-described numerical range, the environmental compatibility is further improved from the viewpoint of carbon neutrality, and the color tone of the polymer obtained using the phenol composition according to the first embodiment is more suitable.

In a case where the content of the above-described phenol (A) is 100% by mass, a content of other components described later is 0.000% by mass. In this case, the content of the other components is equal to or less than the detection lower limit, and may not be detected by quantification.

In the phenol composition according to the first embodiment, it is preferable that the phenol (A) includes at least a structure derived from a benzene ring of a product of one or two or more transformants selected from a transformant HBA-2 (Accession No.: NITE BP-01838) and a transformant HBA-47 (Accession No.: NITE BP-01849). In a case where the above-described phenol (A) includes at least the above-described product, the environmental compatibility is further improved from the viewpoint of carbon neutrality, and the color tone of the polymer obtained using the phenol composition according to the first embodiment is more suitable.

In the phenol composition according to the first embodiment, it is preferable that the phenol (A) includes at least a product of one or two or more transformants selected from a transformant PHE7 (Accession No.: NITE BP-976), a transformant PHE18 (Accession No.: NITE BP-995), a transformant PHE21 (Accession No.: NITE BP-996), and a transformant PHE31 (Accession No.: NITE BP-999). In a case where the above-described phenol (A) includes at least the above-described product, the environmental compatibility is further improved from the viewpoint of carbon neutrality, and the color tone of the polymer obtained using the phenol composition according to the first embodiment is more suitable.

In the phenol composition according to the first embodiment, it is presumed that, in a case where the phenol (A) includes at least a structure derived from a benzene ring of a product of one or two or more transformants selected from the transformant HBA-2 and the transformant HBA-47, or the phenol (A) includes at least a product of one or two or more transformants selected from the transformant PHE7, the transformant PHE18, the transformant PHE21, and the transformant PHE31, the reason why the color tone of the obtained polymer is more suitable is that presence of components present in extremely small amounts, which may adversely affect the color tone, is relatively suppressed.

Here, the components derived from the transformants present in extremely small amounts are extremely large in number, and a content of the components present in extremely small amounts is less than the detection limit and cannot be analyzed. Therefore, in the phenol composition according to the first embodiment, it is considered that there is a so-called "impossible or impractical matter" in which it is impossible to directly specify the feature part of the present aspect by the structure or the property of the object in the aspect in which the phenol (A) includes at least a structure derived from a benzene ring of a product of one or two or more transformants selected from the transformant HBA-2 and the transformant HBA-47, or the aspect in which the phenol (A) includes at least a product of one or two or more transformants selected from the transformant PHE7, the transformant PHE18, the transformant PHE21, and the transformant PHE31.

### (Other components)

In the phenol composition according to the first embodiment, components other than the above-described components may be contained as long as the balance between the environmental compatibility and the color tone of the obtained polymer is not reduced.

Examples of other components include one or two or more selected from the group consisting of the following compounds:
sugars including monosaccharides such as glucose, fructose, mannose, arabinose, xylose, and galactose, disaccharides such as cellobiose, sucrose, lactose, maltose, trehalose, and xylobiose, polysaccharides such as dextrin and soluble starch, and the like;
sugar alcohols such as mannitol, sorbitol, xylitol, and glycerin, organic acids such as acetic acid, citric acid, lactic acid, fumaric acid, maleic acid, and gluconic acid, alcohols such as ethanol and propanol, hydrocarbons such as normal paraffin;
inorganic or organic ammonium compounds such as ammonium chloride, ammonium sulfate, ammonium nitrate, and ammonium acetate, urea, aqueous ammonia, sodium nitrate, potassium nitrate, nitrogen-containing organic compounds such as corn steep liquor, meat extract, peptone, NZ-amine, protein hydrolysate, and amino acid;
potassium dihydrogen phosphate, potassium hydrogen phosphate, magnesium sulfate, sodium chloride, iron(II) sulfate, iron(III) nitrate, manganese sulfate, zinc sulfate, cobalt sulfate, calcium carbonate, and the like; and
solvent such as pure water.

In the phenol composition according to the first embodiment, a content of the above-described other components is preferably equal to or less than 5% by mass, more preferably equal to or less than 4% by mass, still more preferably equal to or less than 3% by mass, even more preferably equal to or less than 2% by mass, and even still more preferably equal to or less than 1% by mass. In a case where the content of the above-described other components is equal to or less than the above-described upper limit value, the color tone of the polymer obtained using the phenol composition according to the first embodiment is more suitable.

In addition, the lower limit value of the content of the above-described other components is not particularly limited, but is, for example, equal to or more than 0.000% by mass. In this case, "0.000% by mass" means that the content of the above-described other components is equal to or less than the detection lower limit, and may not be detected by quantification.

In the phenol composition according to the first embodiment, in a case where a plurality of the above-described other components are contained, the total content of the other components may be equal to or less than the above-described upper limit value and equal to or more than the above-described lower limit value.

Examples of a method for quantifying the contents of the phenol (A) and the other components include high-performance liquid chromatography using a high-performance liquid chromatograph (Chromaster (registered trademark), manufactured by Hitachi High-Tech Science Corporation) equipped with a UV detector, ion chromatography, gas chromatography-mass spectrometry (GC/MS), and inductively coupled plasma-mass spectrometry (ICP-MS).

### [Method for manufacturing phenol composition]

Next, a method for manufacturing the phenol composition according to the first embodiment will be described.

It is preferable that the method for manufacturing the phenol composition according to the first embodiment includes the following steps (A) to (D).
(A) A step of reacting a first transformant and at least one raw material compound selected from the group consisting of a sugar, a compound from which the transformant can generate chorismic acid by metabolism, and chorismic acid and salts thereof, in a reaction intermediate solution containing the first transformant and the at least one raw material compound to generate a precursor
(B) A step of generating a phenol composition from the precursor by a second transformant to obtain a reaction solution containing the phenol composition
(C) A step of removing the transformants in the reaction solution
(D) A step of isolating and concentrating the phenol composition in the reaction solution

### [(A) Step of reacting first transformant and at least one raw material compound selected from the group consisting of sugar, compound from which transformant can generate chorismic acid by metabolism, and chorismic acid and salts thereof, in reaction intermediate solution containing first transformant and at least one raw material compound to generate precursor]

In the method for manufacturing the phenol composition according to the first embodiment, first, the step (A) of reacting a first transformant and at least one raw material compound selected from the group consisting of a sugar, a compound from which the transformant can generate chorismic acid by metabolism, and chorismic acid and salts thereof, in a reaction intermediate solution containing the first transformant and the at least one raw material compound to generate a precursor is performed.

Each compound used in the present step and the reaction conditions will be described below.

### (First transformant)

In the method for manufacturing the phenol composition according to the first embodiment, any transformant can be used as the first transformant as long as the transformant can generate a precursor from a sugar by metabolism. Examples of the precursor in this case include 4-hydroxybenzoic acid. Examples of such a transformant include a coryneform bacterial transformant HBA-2 (Accession No.: NITE BP-01838) described in Japanese Patent No. 6327653 or a coryneform bacterial transformant HBA-47 (Accession No.: NITE BP-01849) described in Japanese Patent No. 6327654.

It is preferable that the first transformant is cultured under aerobic conditions before the reaction. A culture temperature and a culture time are not particularly limited as long as the transformant can be cultured and obtained in an amount equal to or more than a required amount. The culture temperature is, for example, equal to or higher than 25°C and equal to or lower than 38°C. The culture time is, for example, equal to or longer than 12 hours and equal to or shorter than 48 hours.

### (Culture medium)

As a medium used for the aerobic culture of the first transformant before the reaction, a natural medium or a synthetic medium containing a carbon source, a nitrogen source, inorganic salts, and other nutritional substances can be used.

As the carbon source, a sugar (monosaccharides such as glucose, fructose, mannose, xylose, arabinose, and galactose; a disaccharide such as sucrose, maltose, lactose, cellobiose, xylobiose, and trehalose; a polysaccharide such as starch; molasses; and the like), a sugar alcohol such as mannitol, sorbitol, xylitol, and glycerol, an organic acid such as acetic acid, citric acid, lactic acid, fumaric acid, maleic acid, and gluconic acid, an alcohol such as ethanol and propanol, a hydrocarbon such as normal paraffin, and the like can also be used.

The carbon source can be used alone or in combination of two or more kinds thereof. A concentration of the carbon source in the medium is different depending on the compound used, but may be, for example, equal to or more than 0.1 w/v% and equal to or less than 20 w/v%.

As the nitrogen source, inorganic or organic ammonium compounds such as ammonium chloride, ammonium sulfate, ammonium nitrate, and ammonium acetate; urea, aqueous ammonia, sodium nitrate, potassium nitrate; and the like can be used. In addition, nitrogen-containing organic compounds such as corn steep liquor, meat extract, peptone, NZ-amine, protein hydrolysate, and amino acid can also be used. The nitrogen source can be used alone or in combination of two or more kinds thereof. A concentration of the nitrogen source in the medium is different depending on the nitrogen compound used, but may be, for example, equal to or more than 0.1 w/v% and equal to or less than 10 w/v%.

Examples of the inorganic salts include potassium dihydrogen phosphate, potassium hydrogen phosphate, magnesium sulfate, sodium chloride, iron(II) sulfate, iron(III) nitrate, manganese sulfate, zinc sulfate, cobalt sulfate, and calcium carbonate. The inorganic salts can be used alone or in combination of two or more kinds thereof. A concentration of the inorganic salts in the medium is different depending on the inorganic salts used, but may be, for example, equal to or more than 0.01 w/v% and equal to or less than 1 w/v%.

Examples of the nutritional substance include meat extract, peptone, polypeptone, yeast extract, dry yeast, corn steep liquor, skim milk powder, hydrochloric acid hydrolysate of defatted soybean, extracts of animal or plant materials or microbial cells, and decomposition products thereof. A concentration of the nutritional substance in the medium is different depending on the nutritional substance used, but may be, for example, equal to or more than 0.1 w/v% and equal to or less than 10 w/v%.

Furthermore, vitamins can also be added as necessary. Examples of the vitamins include biotin, thiamine (vitamin B1), pyridoxine (vitamin B6), pantothenic acid, inositol, and nicotinic acid.

A pH of the medium is preferably equal to or more than 6 and equal to or less than 8.

Specific examples of a preferred medium include A medium [Inui, M. et al., Metabolic analysis of Corynebacterium glutamicum during lactate and succinate productions under oxygen deprivation conditions. J. Mol. Microbiol. Biotechnol. 7:182-196 (2004)] and BT medium [Oumumasa, C. A. et al., Corynebacterium glutamicum glycerldehyde-3-phosphate dehydrogenase isoforms with opposite, ATP-dependent regulation. J. Mol. Microbiol. Biotechnol. 8:91-103 (2004)]. In these media, a concentration of sugar may be within the above-described range.

### (Raw material compound)

The raw material compound is preferably a compound which can be taken into cells by the first transformant, and more preferably a compound which is easily industrially utilized, such as a compound which is contained in a plant in a large amount.

Among the above-described raw material compounds, as sugars, a sugar which can generate glucose by metabolism of the first transformant is preferable, and glucose is more preferable. Such sugars include an oligosaccharide and a polysaccharide, having a glucose unit. Examples of the above-described oligosaccharide and polysaccharide include monosaccharides such as fructose, mannose, arabinose, xylose, and galactose; disaccharides such as cellobiose, sucrose, lactose, maltose, trehalose, and xylobiose; and polysaccharides such as dextrin and soluble starch. In addition, it is preferable to use glucose from the viewpoint of improving efficiency of generating the precursor and improving manufacturing efficiency of the phenol composition according to the first embodiment.

In addition, examples of the compound from which the first transformant can generate chorismic acid by metabolism, among the above-described raw material compounds, include quinic acid and shikimic acid.

In addition, for example, a raw material derived from a plant can be used as a raw material containing these raw material compounds. As the raw material derived from a plant, for example, molasses or a saccharified solution containing a plurality of sugars such as glucose, which is obtained by saccharifying, with a saccharifying enzyme, non-edible agricultural waste such as straw (rice straw, barley straw, wheat straw, rye straw, oat straw, and the like), bagasse, and corn stover, energy crops such as switchgrass, napier grass, and miscanthus, or wood chips and waste paper, can also be used.

Among these, as the raw material compound, glucose, chorismic acid, quinic acid, or shikimic acid is preferable, and as a raw material containing these compounds, a raw material derived from a plant is preferably used.

### (Reaction intermediate solution)

As the reaction intermediate solution, a natural reaction solution or a synthetic reaction solution, containing a carbon source, a nitrogen source, inorganic salts, and the like, can be used.

As the carbon source, the raw material compound described above or the molasses and the saccharified solution containing the raw material compound may be used. In addition to sugars, as the carbon source, sugar alcohols such as mannitol, sorbitol, xylitol, and glycerin, organic acids such as acetic acid, citric acid, lactic acid, fumaric acid, maleic acid, and gluconic acid, alcohols such as ethanol and propanol, hydrocarbons such as normal paraffin, and the like can also be used.

The carbon source can be used alone or in combination of two or more kinds thereof.

A concentration of the raw material compound in the reaction intermediate solution is preferably equal to or more than 1 w/v% and equal to or less than 20 w/v%, more preferably equal to or more than 2 w/v% and equal to or less than 10 w/v%, and still more preferably equal to or more than 2 w/v% and equal to or less than 5 w/v%.

In addition, a concentration of the total carbon source containing the raw material compound may be equal to or more than 2 w/v% and equal to or less than 5 w/v%.

As the nitrogen source, inorganic or organic ammonium compounds such as ammonium chloride, ammonium sulfate, ammonium nitrate, and ammonium acetate; urea, aqueous ammonia, sodium nitrate, potassium nitrate; and the like can be used. In addition, nitrogen-containing organic compounds such as corn steep liquor, meat extract, peptone, NZ-amine, protein hydrolysate, and amino acid can also be used. The nitrogen source can be used alone or in combination of two or more kinds thereof. A concentration of the nitrogen source in the reaction intermediate solution is different depending on the nitrogen compound used, but may be, for example, equal to or more than 0.1 w/v% and equal to or less than 10 w/v%.

Examples of the inorganic salts include potassium dihydrogen phosphate, potassium hydrogen phosphate, magnesium sulfate, sodium chloride, iron(II) sulfate, iron(III) nitrate, manganese sulfate, zinc sulfate, cobalt sulfate, and calcium carbonate. The inorganic salt can be used alone or in combination of two or more kinds thereof. A concentration of the inorganic salts in the reaction intermediate solution is different depending on the inorganic salt used, but may be, for example, equal to or more than 0.01 w/v% and equal to or less than 1 w/v%.

Examples of the nutritional substance include meat extract, peptone, polypeptone, yeast extract, dry yeast, corn steep liquor, skim milk powder, hydrochloric acid hydrolysate of defatted soybean, extracts of animal or plant materials or microbial cells, and decomposition products thereof. A concentration of the nutritional substance in the reaction intermediate solution is different depending on the nutritional substance used, but may be, for example, equal to or more than 0.1 w/v% and equal to or less than 10 w/v%.

Furthermore, vitamins can also be added as necessary. Examples of the vitamins include biotin, thiamine (vitamin B1), pyridoxine (vitamin B6), pantothenic acid, inositol, and nicotinic acid.

A pH of the reaction intermediate solution is preferably equal to or more than 6 and equal to or less than 8.

Specific examples of a preferred reaction intermediate solution include the A medium and the BT medium described above. In these reaction intermediate solutions, a concentration of sugar may be within the above-described range.

### (Reaction conditions)

From the viewpoint of manufacturing efficiency of the phenol composition, a reaction temperature, that is, a growth temperature of the first transformant is preferably equal to or higher than 20°C, more preferably equal to or higher than 23°C, and still more preferably equal to or higher than 25°C. In addition, from the same viewpoint, the above-described growth temperature is preferably equal to or lower than 50°C, more preferably equal to or lower than 48°C, and still more preferably equal to or lower than 47°C.

From the viewpoint of manufacturing efficiency of the phenol composition, a reaction time is preferably equal to or shorter than 7 days, more preferably equal to or shorter than 5 days, and still more preferably equal to or shorter than 3 days. In addition, the above-described reaction time is not particularly limited as long as the reaction sufficiently proceeds, but is, for example, equal to or longer than 1 day.

The culture may be any of a batch type, a fed-batch type, or a continuous type. Among these, from the viewpoint of manufacturing efficiency of the phenol composition, a batch type is preferable.

The reaction may be performed under aerobic conditions or reducing conditions. Production ability of the phenol composition by the first transformant itself is higher under aerobic conditions. However, in the aerobic conditions, the first transformant proliferates, so that the raw material compound is consumed for the proliferation, and thus the manufacturing efficiency of the phenol composition is reduced.

Therefore, it is preferable to perform the reaction under aerobic conditions and conditions in which the first transformant does not proliferate. In the present specification, "not proliferating" includes substantially not proliferating or hardly proliferating. For example, by using a reaction solution in which one or more of a compound essential for proliferation of a microorganism, such as vitamins including biotin, thiamine, and the like, and a nitrogen source, is deficient or limited, the proliferation of the first transformant can be inhibited or suppressed.

In addition, in the reducing conditions, since the first transformant does not substantially proliferate, the manufacturing efficiency of the precursor is improved because the raw material compound is not consumed for the proliferation, and as a result, the manufacturing efficiency of the phenol composition is increased.

The reducing conditions are defined by a redox potential of the reaction intermediate solution. The redox potential of the reaction intermediate solution is preferably equal to or more than -500 mV and equal to or less than -200 mV, and more preferably equal to or more than -500 mV and equal to or less than -150 mV.

A reduction state of the reaction intermediate solution can be simply estimated by a resazurin indicator (bleaching from blue to colorless in the reduction state), but can be accurately measured using a redox potential difference meter (for example, ORP Electrodes manufactured by Broadley-James Corporation).

As a method for adjusting the reaction intermediate solution under the reducing conditions, any known method can be used without limitation. For example, as a liquid medium of the reaction intermediate solution, an aqueous solution for the reaction intermediate solution may be used instead of distilled water or the like, and a method for adjusting the aqueous solution for the reaction intermediate solution can be referred to, for example, a culture solution adjustment method for an absolute anaerobic microorganism such as a sulfate-reducing microorganism (Pfennig, N. et al., (1981): The dissimilatory sulfate-reducing bacteria, In The Prokaryotes, A Handbook on Habitats Isolation and Identification of Bacteria, Ed. by Starr, M. P. et al., p926-940, Berlin, Springer Verlag.) or "Agricultural Chemistry Experiment Book, Vol. 3, edited by the Department of Agricultural Chemistry, Faculty of Agriculture, Kyoto University, 26th edition, 1990, published by Sangyo Tosho Co., Ltd.", and an aqueous solution under the desired reducing conditions can be obtained.

Specifically, by heating or decompressing distilled water or the like to remove dissolved gas, the aqueous solution for the reaction intermediate solution under reducing conditions can be obtained. In this case, by treating the distilled water or the like under a pressure of preferably equal to or less than 10 mmHg, more preferably equal to or less than 5 mmHg, and still more preferably equal to or less than 3 mmHg, and under conditions of preferably equal to or longer than 1 minute and equal to or shorter than 60 minutes, and more preferably equal to or longer than 5 minutes and equal to or shorter than 40 minutes, the dissolved gas, particularly dissolved oxygen can be removed to prepare the aqueous solution for the reaction intermediate solution under reducing conditions.

In addition, the aqueous solution for the reaction intermediate solution under reducing conditions can be adjusted by adding an appropriate reducing agent (for example, thioglycolic acid, ascorbic acid, cysteine hydrochloride, mercaptoacetic acid, thiolacetic acid, glutathione, sodium sulfide, and the like).

It is also an effective method for adjusting the aqueous solution for the reaction intermediate solution under reducing conditions to appropriately combine these methods.

In a case where the reaction is performed under reducing conditions, it is preferable that the reaction intermediate solution is maintained under reducing conditions during the reaction. In order to maintain the reducing conditions during the reaction, it is desirable to prevent oxygen from the reaction system from being mixed as much as possible; and specific examples thereof include a method of sealing the reaction system with an inert gas such as nitrogen gas and carbon dioxide. As a method for more effectively preventing the oxygen from being mixed, in some cases, it is necessary to appropriately add a pH maintenance adjustment solution to the reaction system or various nutrient dissolution solutions in order to efficiently function the metabolic function in the first transformant of the first embodiment during the reaction; but in such a case, it is effective to remove oxygen from the addition solution in advance.

### [(A)' Step of removing first transformant from precursor]

In addition, in the method for manufacturing the phenol composition according to the first embodiment, from the viewpoint of yield of the phenol composition, the method may include a step (A)' of removing the first transformant from the precursor, after the step (A) of reacting a first transformant and at least one raw material compound selected from the group consisting of a sugar, a compound from which the transformant can generate chorismic acid by metabolism, and chorismic acid and salts thereof, in a reaction intermediate solution containing the first transformant and the at least one raw material compound to generate a precursor and before the step (B) of generating a phenol composition from the precursor by a second transformant to obtain a reaction solution containing the phenol composition, which will be described later.

In the step (A)' of removing the first transformant from the precursor, a method for recovering the reaction solution, which is exemplified in a step (C) of removing the transformant in the reaction solution, which will be described later, can be used. By applying the method for recovering the reaction solution to the precursor, the first transformant can be removed from the precursor to recover the precursor.

### [(B) Step of generating phenol composition from precursor by second transformant to obtain reaction solution containing phenol composition]

Next, the step (B) of generating a phenol composition from the precursor by a second transformant to obtain a reaction solution containing the phenol composition is performed. By culturing as described above, the precursor is produced in the reaction intermediate solution. By adding the second transformant to the reaction intermediate solution, the phenol composition can be generated from the precursor, and a reaction solution containing the phenol composition can be obtained.

Each compound used in the present step and the reaction conditions will be described below.

### (Second transformant)

In the method for manufacturing the phenol composition according to the first embodiment, any transformant can be used as the second transformant as long as the transformant can generate the phenol composition from the precursor. Examples of the precursor in this case include 4-hydroxybenzoic acid. Examples of such a transformant include a coryneform bacterial transformant PHE7 (Accession No.: NITE BP-976) described in Japanese Patent No. 5932649, a coryneform bacterial transformant PHE18 (Accession No.: NITE BP-995) described in Japanese Patent No. 5996434, a coryneform bacterial transformant PHE21 (Accession No.: NITE BP-996) described in Japanese Patent No. 5932660, and a coryneform bacterial transformant PHE31 (Accession No.: NITE BP-999) described in Japanese Patent No. 5887277.

It is preferable that the second transformant is cultured under aerobic conditions before the reaction. A culture temperature and a culture time are not particularly limited as long as the transformant can be cultured and obtained in an amount equal to or more than a required amount. The culture temperature is, for example, equal to or higher than 25°C and equal to or lower than 38°C. The culture time is, for example, equal to or longer than 12 hours and equal to or shorter than 48 hours.

### (Culture medium)

As a medium used for the aerobic culture of the second transformant before the reaction, a natural medium or a synthetic medium containing a carbon source, a nitrogen source, inorganic salts, and other nutritional substances can be used.

As the carbon source, a sugar (monosaccharides such as glucose, fructose, mannose, xylose, arabinose, and galactose; a disaccharide such as sucrose, maltose, lactose, cellobiose, xylobiose, and trehalose; a polysaccharide such as starch; molasses; and the like), a sugar alcohol such as mannitol, sorbitol, xylitol, and glycerol, an organic acid such as acetic acid, citric acid, lactic acid, fumaric acid, maleic acid, and gluconic acid, an alcohol such as ethanol and propanol, a hydrocarbon such as normal paraffin, and the like can also be used.

The carbon source can be used alone or in combination of two or more kinds thereof. A concentration of the carbon source in the medium is different depending on the compound used, but may be, for example, equal to or more than 0.1 w/v% and equal to or less than 20 w/v%.

As the nitrogen source, inorganic or organic ammonium compounds such as ammonium chloride, ammonium sulfate, ammonium nitrate, and ammonium acetate; urea, aqueous ammonia, sodium nitrate, potassium nitrate; and the like can be used. In addition, nitrogen-containing organic compounds such as corn steep liquor, meat extract, peptone, NZ-amine, protein hydrolysate, and amino acid can also be used. The nitrogen source can be used alone or in combination of two or more kinds thereof. A concentration of the nitrogen source in the medium is different depending on the nitrogen compound used, but may be, for example, equal to or more than 0.1 w/v% and equal to or less than 10 w/v%.

Examples of the inorganic salts include potassium dihydrogen phosphate, potassium hydrogen phosphate, magnesium sulfate, sodium chloride, iron(II) sulfate, iron(III) nitrate, manganese sulfate, zinc sulfate, cobalt sulfate, and calcium carbonate. The inorganic salt can be used alone or in combination of two or more kinds thereof. A concentration of the inorganic salts in the medium is different depending on the inorganic salt used, but may be, for example, equal to or more than 0.01 w/v% and equal to or less than 1 w/v%.

Examples of the nutritional substance include meat extract, peptone, polypeptone, yeast extract, dry yeast, corn steep liquor, skim milk powder, hydrochloric acid hydrolysate of defatted soybean, extracts of animal or plant materials or microbial cells, and decomposition products thereof. A concentration of the nutritional substance in the medium is different depending on the nutritional substance used, but may be, for example, equal to or more than 0.1 w/v% and equal to or less than 10 w/v%.

Furthermore, vitamins can also be added as necessary. Examples of the vitamins include biotin, thiamine (vitamin B1), pyridoxine (vitamin B6), pantothenic acid, inositol, and nicotinic acid.

A pH of the medium is preferably equal to or more than 6 and equal to or less than 8.

Specific examples of a preferred medium include A medium [Inui, M. et al., Metabolic analysis of Corynebacterium glutamicum during lactate and succinate productions under oxygen deprivation conditions. J. Mol. Microbiol. Biotechnol. 7:182-196 (2004)] and BT medium [Oumumasa, C. A. et al., Corynebacterium glutamicum glycerldehyde-3-phosphate dehydrogenase isoforms with opposite, ATP-dependent regulation. J. Mol. Microbiol. Biotechnol. 8:91-103 (2004)]. In these media, a concentration of sugar may be within the above-described range.

### (Reaction solution)

As the reaction solution, in addition to the reaction intermediate solution used in the step (A) described above, a buffer solution, an inorganic salt medium, or the like can be further used.

In the reaction solution, the precursor may be a salt such as a sodium salt and a potassium salt, or an ester with an alcohol having 1 to 4 carbon atoms. Among these, a salt is preferable, and a sodium salt is more preferable. The precursor can be used alone or in combination of two or more kinds thereof.

A concentration of the precursor in the reaction solution is preferably equal to or more than 1 w/v% and equal to or less than 20 w/v%, more preferably equal to or more than 2 w/v% and equal to or less than 10 w/v%, and still more preferably equal to or more than 2 w/v% and equal to or less than 5 w/v%. In a case where the concentration of the precursor is within the above-described numerical range, the manufacturing efficiency of the phenol composition is further improved.

Examples of the buffer solution include a phosphate buffer, a Tris buffer, and a carbonate buffer. From the viewpoint of manufacturing efficiency of the phenol composition, a concentration of the buffer solution is preferably equal to or more than 10 mM and equal to or less than 150 mM.

Examples of the inorganic salt medium include a medium containing one or two or more kinds of inorganic salts such as potassium dihydrogen phosphate, potassium hydrogen phosphate, magnesium sulfate, sodium chloride, iron(III) nitrate, manganese sulfate, zinc sulfate, cobalt sulfate, and calcium carbonate. Among these, a medium containing magnesium sulfate is preferable. Specific examples of the inorganic salt medium include A medium [Inui, M. et al., Metabolic analysis of Corynebacterium glutamicum during lactate and succinate productions under oxygen deprivation conditions. J. Mol. Microbiol. Biotechnol. 7:182-196 (2004)] and BT medium [Oumumasa, C. A. et al., Corynebacterium glutamicum glycerldehyde-3-phosphate dehydrogenase isoforms with opposite, ATP-dependent regulation. J. Mol. Microbiol. Biotechnol. 8:91-103 (2004)]. A concentration of the inorganic salts in the medium is different depending on the inorganic salt used, but may be usually equal to or more than 0.01 w/v% and equal to or less than 1 w/v%.

A pH of the reaction solution is preferably equal to or more than 6 and equal to or less than 8. During the reaction, it is preferable to react while controlling the pH of the reaction solution to be neutral, particularly 7 with a pH controller (for example, model number: DT-1023, manufactured by Able Co., Ltd.) using an aqueous ammonia solution, a sodium hydroxide aqueous solution, or the like.

### (Reaction conditions)

From the viewpoint of manufacturing efficiency of the phenol composition, a reaction temperature, that is, a growth temperature of the second transformant is preferably equal to or higher than 20°C, more preferably equal to or higher than 23°C, and still more preferably equal to or higher than 25°C. In addition, from the same viewpoint, the above-described growth temperature is preferably equal to or lower than 50°C, more preferably equal to or lower than 48°C, and still more preferably equal to or lower than 47°C.

From the viewpoint of manufacturing efficiency of the phenol composition, a reaction time is preferably equal to or shorter than 7 days, more preferably equal to or shorter than 5 days, and still more preferably equal to or shorter than 3 days. In addition, the above-described reaction time is not particularly limited as long as the reaction sufficiently proceeds, but is, for example, equal to or longer than 1 day.

The culture may be any of a batch type, a fed-batch type, or a continuous type. Among these, from the viewpoint of manufacturing efficiency of the phenol composition, a batch type is preferable.

The reaction may be performed under aerobic conditions, but is preferably performed under reducing conditions. In the reducing conditions, since the second transformant does not proliferate, the manufacturing efficiency of the phenol composition is increased. In the present specification, "not proliferating" includes substantially not proliferating or hardly proliferating.

The reducing conditions are defined by a redox potential of the reaction solution. From the viewpoint of manufacturing efficiency of the phenol composition, a redox potential of the reaction intermediate solution is preferably equal to or more than -500 mV and equal to or more than -200 mV, and more preferably equal to or more than -500 mV and equal to or less than -150 mV.

A reduction state of the reaction solution can be simply estimated by a resazurin indicator (bleaching from blue to colorless in the reduction state), but can be accurately measured using a redox potential difference meter (for example, ORP Electrodes manufactured by Broadley-James Corporation).

As a method for adjusting the reaction solution under the reducing conditions, any known method can be used without limitation. For example, as a liquid medium of the reaction solution, an aqueous solution for the reaction solution may be used instead of distilled water or the like, and a method for adjusting the aqueous solution for the reaction solution can be referred to, for example, a culture solution adjustment method for an absolute anaerobic microorganism such as a sulfate-reducing microorganism (Pfennig, N. et al., (1981): The dissimilatory sulfate-reducing bacteria, In The Prokaryotes, A Handbook on Habitats Isolation and Identification of Bacteria, Ed. by Starr, M. P. et al., p926-940, Berlin, Springer Verlag.) or "Agricultural Chemistry Experiment Book, Vol. 3, edited by the Department of Agricultural Chemistry, Faculty of Agriculture, Kyoto University, 26th edition, 1990, published by Sangyo Tosho Co., Ltd.", and an aqueous solution under the desired reducing conditions can be obtained.

Specifically, by heating or decompressing distilled water or the like to remove dissolved gas, the aqueous solution for the reaction solution under reducing conditions can be obtained. In this case, by treating the distilled water or the like under a pressure of preferably equal to or less than 10 mmHg, more preferably equal to or less than 5 mmHg, and still more preferably equal to or less than 3 mmHg, and under conditions of preferably equal to or longer than 1 minute and equal to or shorter than 60 minutes, and more preferably equal to or longer than 5 minutes and equal to or shorter than 40 minutes, the dissolved gas, particularly dissolved oxygen can be removed to prepare the aqueous solution for the reaction solution under reducing conditions.

In addition, the aqueous solution for the reaction solution under reducing conditions can be adjusted by adding an appropriate reducing agent (for example, thioglycolic acid, ascorbic acid, cysteine hydrochloride, mercaptoacetic acid, thiolacetic acid, glutathione, sodium sulfide, and the like).

It is also an effective method for adjusting the aqueous solution for the reaction solution under reducing conditions to appropriately combine these methods.

In a case where the reaction is performed under reducing conditions, it is preferable that the reaction solution is maintained under reducing conditions during the reaction. In order to maintain the reducing conditions during the reaction, it is desirable to prevent oxygen from the reaction system from being mixed as much as possible; and specific examples thereof include a method of sealing the reaction system with an inert gas such as nitrogen gas and carbon dioxide. As a method for more effectively preventing the oxygen from being mixed, in some cases, it is necessary to appropriately add a pH maintenance adjustment solution to the reaction system or various nutrient dissolution solutions in order to efficiently function the metabolic function in the second transformant of the first embodiment during the reaction; but in such a case, it is effective to remove oxygen from the addition solution in advance.

### [(C) Step of removing transformants in reaction solution]

Next, the step (C) of removing the transformants in the reaction solution is performed. By culturing as described above, phenol is produced in the reaction solution, but the transformants remain in the reaction solution. By removing the transformants, the reaction solution containing the phenol composition can be recovered.

Examples of a method for recovering the reaction solution include a method of isolating the reaction solution and the transformants. In this case, the above-described step (A)' of removing the first transformant from the precursor may be performed to isolately isolate the first transformant and the second transformant from the reaction solution; or the step (A)' of removing the first transformant from the precursor may not be performed and the first transformant and the second transformant simultaneously isolated from the reaction solution.

As a method of isolating the transformants, a known isolating method can be used. Examples of the known isolating method include collection of a supernatant liquid by standing, membrane filtration, and centrifugal separation. These methods can be appropriately combined and performed.

As a treatment condition by the membrane filtration, the treatment can be performed under general filtration conditions. From the viewpoint of improving the manufacturing efficiency of the phenol composition, a membrane pore diameter is preferably equal to or more than 0.01 µm, more preferably equal to or more than 0.03 µm, still more preferably equal to or more than 0.05 µm, even more preferably equal to or more than 0.07 µm, even still more preferably equal to or more than 0.10 µm, further more preferably equal to or more than 0.15 µm, and even further more preferably equal to or more than 0.20 µm. In addition, from the same viewpoint, the membrane pore diameter is preferably equal to or less than 3 µm, more preferably equal to or less than 2 µm, and still more preferably equal to or less than 1 µm. Examples of a method of measuring the membrane pore diameter include a general measurement method using a mercury intrusion method, a bubble point test, a bacterial filtration method, and the like; but it is preferable to use a value obtained by the bubble point test. Examples of a material of the membrane used for the membrane filtration include a polymer membrane, a ceramic membrane, and a stainless steel membrane.

As a centrifuge used for the centrifugal separation, general equipment such as a isolating plate type, a cylindrical type, and a decanter type can be used. From the viewpoint of improving the manufacturing efficiency of the phenol composition, a lower limit value of a temperature during the centrifugal separation is preferably equal to or higher than 0°C, more preferably equal to or higher than 1°C, and still more preferably equal to or higher than 3°C. In addition, from the same viewpoint, the upper limit value of the temperature during the centrifugal separation is preferably equal to or lower than 70°C, more preferably equal to or lower than 50°C, still more preferably equal to or lower than 30°C, even more preferably equal to or lower than 10°C, and even still more preferably equal to or lower than 5°C.

In addition, a relative centrifugal force and a time can be appropriately set, but from the viewpoint of improving the polymerizability of the phenol composition, the lower limit value of the relative centrifugal force is preferably equal to or more than 3,000 g, more preferably equal to or more than 5,000 g, still more preferably equal to or more than 7,000 g, even more preferably equal to or more than 10,000 g, and even still more preferably equal to or more than 15,000 g. In addition, from the viewpoint of improving the manufacturing efficiency of the phenol composition, the upper limit value of the relative centrifugal force is preferably equal to or less than 50,000 g, more preferably equal to or less than 25,000 g, and still more preferably equal to or less than 20,000 g.

Similarly, from the viewpoint of improving the color tone of the polymer obtained using the phenol composition, the lower limit value of the time is preferably equal to or longer than 0.2 minutes, more preferably equal to or longer than 0.5 minutes, still more preferably equal to or longer than 1 minute, and even more preferably equal to or longer than 5 minutes. In addition, from the viewpoint of improving the manufacturing efficiency of the phenol composition, the upper limit value of the time is preferably equal to or shorter than 75 minutes, more preferably equal to or shorter than 60 minutes, still more preferably equal to or shorter than 30 minutes, even more preferably equal to or shorter than 15 minutes, and even still more preferably equal to or shorter than 10 minutes.

### [(D) Step of isolating and concentrating phenol composition in reaction solution]

Finally, the step (D) of isolating and concentrating the phenol composition in the reaction solution is performed. In the first embodiment, the phenol composition in the reaction solution can be recovered as described above, but the phenol composition is isolated and concentrated from the reaction solution by a known method in order to improve the phenol concentration in the phenol composition and further improve the color tone of the obtained polymer. Examples of such a known method include a distillation method, a pervaporation method, a membrane permeation method, and an organic solvent extraction method. These methods can be appropriately combined and performed.

As the distillation method, any known distillation method can be used. The known distillation method is not particularly limited, and a normal pressure distillation method using a distillation column, a vacuum distillation method using an evaporator, a steam distillation method, a thin film distillation method, and the like can be used.

The pervaporation method refers to a method of obtaining a phenol composition in a gaseous state by permeating through a pervaporation membrane. The pervaporation membrane has a function of preferentially permeating phenol over water. Therefore, the phenol composition in the reaction solution preferentially permeates, over water, the pervaporation membrane from the supply side space to move to the permeation side space. As a result, the phenol composition obtained in the permeation side space has a higher phenol concentration than the reaction solution. As described above, by using the pervaporation method, a phenol composition in a vaporized state can be obtained from the reaction solution, and the phenol concentration in the obtained phenol composition can be higher than that in the reaction solution.

A shape of the pervaporation membrane is not particularly limited, and for example, a dense membrane of a bag type, a flat membrane type, a hollow fiber type, a tubular type, a spiral type, or the like can be adopted.

An average thickness of such a pervaporation membrane is not particularly limited, but is preferably approximately 10 to 500 µm and more preferably approximately 20 to 400 µm. By setting the average thickness of the pervaporation membrane to be within the above-described range, the supply side space and the permeation side space can be reliably separated, and thus the phenol composition in a vaporized state can be more reliably obtained from the reaction solution.

In addition, a material of the pervaporation membrane is not particularly limited, and examples thereof include an inorganic membrane such as a DDR-type zeolite membrane, a polymer membrane such as polyimide, polyamide, polysulfone, polyethersulfone, and polyamideimide, and a composite membrane thereof. Among these, an inorganic membrane is preferable, and a DDR-type zeolite membrane is more preferable. As a result, mechanical characteristics of the pervaporation membrane can be improved while improving the isolating performance of phenol.

### [Use]

The phenol composition according to the first embodiment can be used for the same applications as a phenol composition synthesized by a petroleum chemical process in the related art, using a fossil resource as a raw material. That is, examples of the application of the phenol composition according to the first embodiment include a precursor of bisphenol A, a raw material of a polymer, an industrial chemical, and a fiber.

Among these, it is preferable to use for a polymer application. That is, the polymer using the phenol composition according to the first embodiment is a polymer containing at least a structural unit derived from the phenol (A) in the phenol composition according to the first embodiment.

The polymer according to the first embodiment may be any polymer as long as the polymer requires the phenol for synthesis. Since the phenol composition according to the first embodiment is improved in the balance between the environmental compatibility and the color tone of the obtained polymer, the polymer according to the first embodiment is preferably a phenolic resin.

### <Second embodiment>

### [4-Hydroxybenzoic acid composition]

The 4-hydroxybenzoic acid composition according to the second embodiment contains at least 4-hydroxybenzoic acid (A) in which an actually measured biobased content representing a proportion of radioactive carbon ¹⁴C derived from a biomass resource, which is determined based on a concentration of radioactive carbon in circulating carbon in the 1950s as measured in accordance with ASTM D6866-20, is equal to or more than 80% and equal to or less than 100%, and a content of shikimic acid is equal to or less than 0.012% by mass with respect to 100% by mass of the entire 4-hydroxybenzoic acid composition.

In such a 4-hydroxybenzoic acid composition, the actually measured biobased content indicates the amount of carbon derived from a plant in the 4-hydroxybenzoic acid (A); and as the value is larger, the 4-hydroxybenzoic acid composition is composed of a natural raw material. Therefore, as the actually measured biobased content is larger, environmental compatibility is favorable from the viewpoint of carbon neutrality. In addition, in a case where the content of shikimic acid is equal to or less than 0.012% by mass, it is possible to obtain a 4-hydroxybenzoic acid composition in which the polymerizability is more suitable.

Such a 4-hydroxybenzoic acid composition can be produced by a manufacturing method described later, particularly by (i) using a specific raw material derived from a plant as a raw material compound, (ii) removing impurities by adding an adsorbent to a reaction solution, and (iii) washing the reaction solution with pure water during the recovery.

In the 4-hydroxybenzoic acid (A) of the 4-hydroxybenzoic acid composition according to the second embodiment, the actually measured biobased content representing the proportion of radioactive carbon ¹⁴C derived from a biomass resource, which is determined based on a concentration of radioactive carbon in circulating carbon in the 1950s as measured in accordance with ASTM D6866-20, is equal to or more than 80% and equal to or less than 100%, preferably equal to or more than 85% and equal to or less than 100%, more preferably equal to or more than 90% and equal to or less than 100%, and still more preferably equal to or more than 95% and equal to or less than 100%. In a case where the above-described actually measured biobased content is within the above-described numerical range, the environmental compatibility is further improved from the viewpoint of carbon neutrality.

In this case, the actually measured biobased content representing the proportion of radioactive carbon ¹⁴C in all carbon atoms in the 4-hydroxybenzoic acid (A) of the 4-hydroxybenzoic acid composition is calculated from a content of radioactive carbon ¹⁴C in a sample measured based on a biobased concentration test specification ASTM D6866-20 defined by American Society for Testing and Materials (ASTM), and a concentration of the radioactive carbon ¹⁴C in circulating carbon in the 1950s. A plurality of analysis methods are specified in the test specification, and among these, an analysis method B (AMS method) is preferably used. For example, an analysis device such as an accelerator mass spectrometer Pelletron AMS manufactured by NEC Corporation is used for the analysis method B.

In the 4-hydroxybenzoic acid composition according to the second embodiment, the upper limit value of the content of shikimic acid is equal to or less than 0.012% by mass, preferably equal to or less than 0.008% by mass, more preferably equal to or less than 0.006% by mass, still more preferably equal to or less than 0.003% by mass, and even more preferably equal to or less than 0.001% by mass, with respect to 100% by mass of the entire 4-hydroxybenzoic acid composition. In a case where the content of shikimic acid is equal to or less than the above-described upper limit value, the polymerizability of the 4-hydroxybenzoic acid composition according to the second embodiment is more suitable.

In addition, the lower limit value of the content of shikimic acid is not particularly limited, but is, for example, equal to or more than 0.000% by mass. In this case, "0.000% by mass" means that the content of shikimic acid is equal to or less than the detection lower limit, and may not be detected by quantification.

In the 4-hydroxybenzoic acid composition according to the second embodiment, a content of the above-described 4-hydroxybenzoic acid (A) is preferably equal to or more than 95% by mass and equal to or less than 100% by mass, more preferably equal to or more than 96% by mass and equal to or less than 100% by mass, still more preferably equal to or more than 97% by mass and equal to or less than 100% by mass, even more preferably equal to or more than 98% by mass and equal to or less than 100% by mass, and even still more preferably equal to or more than 99% by mass and equal to or less than 100% by mass, with respect to 100% by mass of the entire 4-hydroxybenzoic acid composition. In a case where the content of the above-described 4-hydroxybenzoic acid (A) is within the above-described numerical range, the environmental compatibility is further improved from the viewpoint of carbon neutrality, and the polymerizability of the 4-hydroxybenzoic acid composition according to the second embodiment is more suitable.

In a case where the content of the above-described 4-hydroxybenzoic acid (A) is 100% by mass, the content of shikimic acid described above and a content of other components described later are 0.000% by mass. In this case, the contents of shikimic acid and the other components are equal to or less than the detection lower limit, and may not be detected by quantification.

In the 4-hydroxybenzoic acid composition according to the second embodiment, it is preferable that the 4-hydroxybenzoic acid (A) includes at least a product of one or two transformants selected from a transformant HBA-2 (Accession No.: NITE BP-01838) and a transformant HBA-47 (Accession No.: NITE BP-01849). In a case where the 4-hydroxybenzoic acid (A) includes at least the above-described product, the environmental compatibility is further improved from the viewpoint of carbon neutrality, and the polymerizability of the 4-hydroxybenzoic acid composition according to the second embodiment is more suitable.

In the 4-hydroxybenzoic acid composition according to the second embodiment, it is presumed that, in a case where the 4-hydroxybenzoic acid (A) includes at least a product of one or two or more transformants selected from the transformant HBA-2 and the transformant HBA-47, the reason why the polymerizability is more suitable is that presence of components present in extremely small amounts, which may adversely affect the polymerizability, is relatively suppressed.

Here, the components derived from the transformants present in extremely small amounts are extremely large in number, and a content of the components present in extremely small amounts is less than the detection limit and cannot be analyzed. Therefore, in the 4-hydroxybenzoic acid composition according to the second embodiment, it is considered that there is a so-called "impossible or impractical matter" in which it is impossible to directly specify the feature part of the present aspect by the structure or the property of the object in the aspect in which the 4-hydroxybenzoic acid (A) includes at least a product of one or two or more transformants selected from the transformant HBA-2 and the transformant HBA-47.

### (Other components)

In the 4-hydroxybenzoic acid composition according to the second embodiment, components other than the above-described components may be contained as long as the balance between the environmental compatibility and the polymerizability is not reduced.

Examples of other components include one or two or more selected from the group consisting of the following compounds:
sugars including monosaccharides such as glucose, fructose, mannose, arabinose, xylose, and galactose, disaccharides such as cellobiose, sucrose, lactose, maltose, trehalose, and xylobiose, polysaccharides such as dextrin and soluble starch, and the like;
sugar alcohols such as mannitol, sorbitol, xylitol, and glycerin, organic acids such as acetic acid, citric acid, lactic acid, fumaric acid, maleic acid, and gluconic acid, alcohols such as ethanol and propanol, hydrocarbons such as normal paraffin;
inorganic or organic ammonium compounds such as ammonium chloride, ammonium sulfate, ammonium nitrate, and ammonium acetate, urea, aqueous ammonia, sodium nitrate, potassium nitrate, nitrogen-containing organic compounds such as corn steep liquor, meat extract, peptone, NZ-amine, protein hydrolysate, and amino acid; and
potassium dihydrogen phosphate, potassium hydrogen phosphate, magnesium sulfate, sodium chloride, iron(II) sulfate, iron(III) nitrate, manganese sulfate, zinc sulfate, cobalt sulfate, calcium carbonate, and the like.

In the 4-hydroxybenzoic acid composition according to the second embodiment, a content of the above-described other components is preferably equal to or less than 5% by mass, more preferably equal to or less than 4% by mass, still more preferably equal to or less than 3% by mass, even more preferably equal to or less than 2% by mass, and even still more preferably equal to or less than 1% by mass. In a case where the content of the above-described other components is equal to or less than the above-described upper limit value, the polymerizability of the 4-hydroxybenzoic acid composition according to the second embodiment is more suitable.

In addition, the lower limit value of the content of the above-described other components is not particularly limited, but is, for example, equal to or more than 0.000% by mass. In this case, "0.000% by mass" means that the content of the above-described other components is equal to or less than the detection lower limit, and may not be detected by quantification.

In the 4-hydroxybenzoic acid composition according to the second embodiment, in a case where a plurality of the above-described other components are contained, the total content of the other components may be equal to or less than the above-described upper limit value and equal to or more than the above-described lower limit value.

Examples of a method for quantifying the contents of shikimic acid, the 4-hydroxybenzoic acid (A), and the other components include high-performance liquid chromatography using a high-performance liquid chromatograph (Chromaster (registered trademark), manufactured by Hitachi High-Tech Science Corporation) equipped with a UV detector, ion chromatography, gas chromatography-mass spectrometry (GC/MS), and inductively coupled plasma-mass spectrometry (ICP-MS).

A method for manufacturing the 4-hydroxybenzoic acid composition according to the second embodiment will be described later.

### <Third embodiment>

### [4-Hydroxybenzoic acid composition]

The 4-hydroxybenzoic acid composition according to the third embodiment contains at least 4-hydroxybenzoic acid (A) in which an actually measured biobased content representing a proportion of radioactive carbon ¹⁴C derived from a biomass resource, which is determined based on a concentration of radioactive carbon in circulating carbon in the 1950s as measured in accordance with ASTM D6866-20, is equal to or more than 80% and equal to or less than 100%, and an absorbance at a wavelength of 425 nm when the aromatic compound composition is dissolved in ethanol at a concentration of 20 g/L is equal to or less than 0.015.

In such a 4-hydroxybenzoic acid composition, the actually measured biobased content indicates the amount of carbon derived from a plant in the 4-hydroxybenzoic acid (A); and as the value is larger, the 4-hydroxybenzoic acid composition is composed of a natural raw material. Therefore, as the actually measured biobased content is larger, environmental compatibility is favorable from the viewpoint of carbon neutrality. In addition, in a case where the above-described absorbance is equal to or less than 0.015, it is possible to obtain a 4-hydroxybenzoic acid composition in which the color tone is more suitable even in a case where a raw material derived from a plant is used.

Such a 4-hydroxybenzoic acid composition can be produced by a manufacturing method described later, particularly by (i) using a specific raw material derived from a plant as a raw material compound, (ii) removing impurities by adding an adsorbent to a reaction solution, and (iii) washing the reaction solution with pure water during the recovery.

In the 4-hydroxybenzoic acid (A) of the 4-hydroxybenzoic acid composition according to the third embodiment, the actually measured biobased content representing the proportion of radioactive carbon ¹⁴C derived from a biomass resource, which is determined based on a concentration of radioactive carbon in circulating carbon in the 1950s as measured in accordance with ASTM D6866-20, is equal to or more than 80% and equal to or less than 100%, preferably equal to or more than 85% and equal to or less than 100%, more preferably equal to or more than 90% and equal to or less than 100%, and still more preferably equal to or more than 95% and equal to or less than 100%. In a case where the above-described actually measured biobased content is within the above-described numerical range, the environmental compatibility is further improved from the viewpoint of carbon neutrality.

In this case, the actually measured biobased content representing the proportion of radioactive carbon ¹⁴C in all carbon atoms in the 4-hydroxybenzoic acid (A) of the 4-hydroxybenzoic acid composition is calculated from a content of radioactive carbon ¹⁴C in a sample measured based on a biobased concentration test specification ASTM D6866-20 defined by American Society for Testing and Materials (ASTM), and a concentration of the radioactive carbon ¹⁴C in circulating carbon in the 1950s. A plurality of analysis methods are specified in the test specification, and among these, an analysis method B (AMS method) is preferably used. For example, an analysis device such as an accelerator mass spectrometer Pelletron AMS manufactured by NEC Corporation is used for the analysis method B.

In the 4-hydroxybenzoic acid composition according to the third embodiment, the upper limit value of the absorbance at a wavelength of 425 nm when the 4-hydroxybenzoic acid composition is dissolved in ethanol at a concentration of 20 g/L is equal to or less than 0.015, preferably equal to or less than 0.013, more preferably equal to or less than 0.010, and still more preferably equal to or less than 0.006. In a case where the absorbance at a wavelength of 425 nm is equal to or less than the above-described upper limit value, the color tone of the 4-hydroxybenzoic acid composition according to the third embodiment is more suitable.

In addition, the lower limit value of the absorbance at a wavelength of 425 nm when the 4-hydroxybenzoic acid composition is dissolved in ethanol at a concentration of 20 g/L is not particularly limited, but is, for example, equal to or more than 0.000, and from the viewpoint of environmental compatibility and manufacturing efficiency, it is preferably equal to or more than 0.001, and may be equal to or more than 0.003. In this case, the absorbance of 0.000 means that the absorbance at a wavelength of 425 nm is equal to or less than the detection lower limit, and may not be detected by quantification.

In the 4-hydroxybenzoic acid composition according to the third embodiment, a content of the above-described 4-hydroxybenzoic acid (A) is preferably equal to or more than 95% by mass and equal to or less than 100% by mass, more preferably equal to or more than 96% by mass and equal to or less than 100% by mass, still more preferably equal to or more than 97% by mass and equal to or less than 100% by mass, even more preferably equal to or more than 98% by mass and equal to or less than 100% by mass, and even still more preferably equal to or more than 99% by mass and equal to or less than 100% by mass, with respect to 100% by mass of the entire 4-hydroxybenzoic acid composition. In a case where the content of the above-described 4-hydroxybenzoic acid (A) is within the above-described numerical range, the environmental compatibility is further improved from the viewpoint of carbon neutrality, and the color tone of the 4-hydroxybenzoic acid composition according to the third embodiment is more suitable.

In a case where the content of the above-described 4-hydroxybenzoic acid (A) is 100% by mass, a content of other components described later is 0.000% by mass. In this case, the content of the other components is equal to or less than the detection lower limit, and may not be detected by quantification.

In the 4-hydroxybenzoic acid composition according to the third embodiment, it is preferable that the 4-hydroxybenzoic acid (A) includes at least a product of one or two transformants selected from a transformant HBA-2 (Accession No.: NITE BP-01838) and a transformant HBA-47 (Accession No.: NITE BP-01849). In a case where the 4-hydroxybenzoic acid (A) includes at least the above-described product, the environmental compatibility is further improved from the viewpoint of carbon neutrality, and the color tone of the 4-hydroxybenzoic acid composition according to the third embodiment is more suitable.

In the 4-hydroxybenzoic acid composition according to the third embodiment, it is presumed that, in a case where the 4-hydroxybenzoic acid (A) includes at least a product of one or two or more transformants selected from the transformant HBA-2 and the transformant HBA-47, the reason why the color tone is more suitable is that presence of components present in extremely small amounts, which may adversely affect the color tone, is relatively suppressed.

Here, the components derived from the transformants present in extremely small amounts are extremely large in number, and a content of the components present in extremely small amounts is less than the detection limit and cannot be analyzed. Therefore, in the 4-hydroxybenzoic acid composition according to the third embodiment, it is considered that there is a so-called "impossible or impractical matter" in which it is impossible to directly specify the feature part of the present aspect by the structure or the property of the object in the aspect in which the 4-hydroxybenzoic acid (A) includes at least a product of one or two or more transformants selected from the transformant HBA-2 and the transformant HBA-47.

### (Other components)

In the 4-hydroxybenzoic acid composition according to the third embodiment, components other than the above-described components may be contained as long as the balance between the environmental compatibility and the color tone is not reduced.

Examples of other components include one or two or more selected from the group consisting of the following compounds:
sugars including monosaccharides such as glucose, fructose, mannose, arabinose, xylose, and galactose, disaccharides such as cellobiose, sucrose, lactose, maltose, trehalose, and xylobiose, polysaccharides such as dextrin and soluble starch, and the like;
sugar alcohols such as mannitol, sorbitol, xylitol, and glycerin, organic acids such as acetic acid, citric acid, lactic acid, fumaric acid, maleic acid, and gluconic acid, alcohols such as ethanol and propanol, hydrocarbons such as normal paraffin;
inorganic or organic ammonium compounds such as ammonium chloride, ammonium sulfate, ammonium nitrate, and ammonium acetate, urea, aqueous ammonia, sodium nitrate, potassium nitrate, nitrogen-containing organic compounds such as corn steep liquor, meat extract, peptone, NZ-amine, protein hydrolysate, and amino acid; and
potassium dihydrogen phosphate, potassium hydrogen phosphate, magnesium sulfate, sodium chloride, iron(II) sulfate, iron(III) nitrate, manganese sulfate, zinc sulfate, cobalt sulfate, calcium carbonate, and the like.

In the 4-hydroxybenzoic acid composition according to the third embodiment, a content of the above-described other components is preferably equal to or less than 5% by mass, more preferably equal to or less than 4% by mass, still more preferably equal to or less than 3% by mass, even more preferably equal to or less than 2% by mass, and even still more preferably equal to or less than 1% by mass. In a case where the content of the above-described other components is equal to or less than the above-described upper limit value, the color tone of the 4-hydroxybenzoic acid composition according to the third embodiment is more suitable.

In addition, the lower limit value of the content of the above-described other components is not particularly limited, but is, for example, equal to or more than 0.000% by mass. In this case, "0.000% by mass" means that the content of the above-described other components is equal to or less than the detection lower limit, and may not be detected by quantification.

In the 4-hydroxybenzoic acid composition according to the third embodiment, in a case where a plurality of the above-described other components are contained, the total content of the other components may be equal to or less than the above-described upper limit value and equal to or more than the above-described lower limit value.

Examples of a method for quantifying the contents of the 4-hydroxybenzoic acid (A) and the other components include high-performance liquid chromatography using a high-performance liquid chromatograph (Chromaster (registered trademark), manufactured by Hitachi High-Tech Science Corporation) equipped with a UV detector, ion chromatography, gas chromatography-mass spectrometry (GC/MS), and inductively coupled plasma-mass spectrometry (ICP-MS).

### [Method for manufacturing 4-hydroxybenzoic acid composition]

Next, a method for manufacturing the 4-hydroxybenzoic acid composition according to the second embodiment and the third embodiment will be described.

It is preferable that the method for manufacturing the 4-hydroxybenzoic acid composition according to the second embodiment and the third embodiment includes the following steps (A) to (C).
(A) A step of reacting a transformant and at least one raw material compound selected from the group consisting of a sugar, a compound from which the transformant can generate chorismic acid by metabolism, and chorismic acid and salts thereof, in a reaction solution containing the transformant and the at least one raw material compound
(B) A step of removing impurities in the reaction solution
(C) A step of recovering the 4-hydroxybenzoic acid composition in the reaction solution.

### [(A) Step of reacting transformant and at least one raw material compound selected from the group consisting of sugar, compound from which transformant can generate chorismic acid by metabolism, and chorismic acid and salts thereof, in reaction solution containing transformant and at least one raw material compound]

In the method for manufacturing the 4-hydroxybenzoic acid composition according to the second embodiment and the third embodiment, first, the step (A) of reacting a transformant and at least one raw material compound selected from the group consisting of a sugar, a compound from which the transformant can generate chorismic acid by metabolism, and chorismic acid and salts thereof, in a reaction solution containing the transformant and the at least one raw material compound is performed.

Each compound used in the present step and the reaction conditions will be described below.

### (Transformant)

In the method for manufacturing the 4-hydroxybenzoic acid composition according to the second embodiment and the third embodiment, any transformant can be used as the transformant as long as the transformant can generate 4-hydroxybenzoic acid from a sugar by metabolism. Examples of such a transformant include a coryneform bacterial transformant HBA-2 (Accession No.: NITE BP-01838) described in Japanese Patent No. 6327653 or a coryneform bacterial transformant HBA-47 (Accession No.: NITE BP-01849) described in Japanese Patent No. 6327654.

It is preferable that the transformant is cultured under aerobic conditions before the reaction. A culture temperature and a culture time are not particularly limited as long as the transformant can be cultured and obtained in an amount equal to or more than a required amount. The culture temperature is, for example, equal to or higher than 25°C and equal to or lower than 38°C. The culture time is, for example, equal to or longer than 12 hours and equal to or shorter than 48 hours.

### (Culture medium)

As a medium used for the aerobic culture of the transformant before the reaction, a natural medium or a synthetic medium containing a carbon source, a nitrogen source, inorganic salts, and other nutritional substances can be used.

As the carbon source, a sugar (monosaccharides such as glucose, fructose, mannose, xylose, arabinose, and galactose; a disaccharide such as sucrose, maltose, lactose, cellobiose, xylobiose, and trehalose; a polysaccharide such as starch; molasses; and the like), a sugar alcohol such as mannitol, sorbitol, xylitol, and glycerol, an organic acid such as acetic acid, citric acid, lactic acid, fumaric acid, maleic acid, and gluconic acid, an alcohol such as ethanol and propanol, a hydrocarbon such as normal paraffin, and the like can also be used.

The carbon source can be used alone or in combination of two or more kinds thereof. A concentration of the carbon source in the medium is different depending on the compound used, but may be, for example, equal to or more than 0.1 w/v% and equal to or less than 20 w/v%.

As the nitrogen source, inorganic or organic ammonium compounds such as ammonium chloride, ammonium sulfate, ammonium nitrate, and ammonium acetate; urea, aqueous ammonia, sodium nitrate, potassium nitrate; and the like can be used. In addition, nitrogen-containing organic compounds such as corn steep liquor, meat extract, peptone, NZ-amine, protein hydrolysate, and amino acid can also be used. The nitrogen source can be used alone or in combination of two or more kinds thereof. A concentration of the nitrogen source in the medium is different depending on the nitrogen compound used, but may be, for example, equal to or more than 0.1 w/v% and equal to or less than 10 w/v%.

Examples of the inorganic salts include potassium dihydrogen phosphate, potassium hydrogen phosphate, magnesium sulfate, sodium chloride, iron(II) sulfate, iron(III) nitrate, manganese sulfate, zinc sulfate, cobalt sulfate, and calcium carbonate. The inorganic salts can be used alone or in combination of two or more kinds thereof. A concentration of the inorganic salts in the medium is different depending on the inorganic salts used, but may be, for example, equal to or more than 0.01 w/v% and equal to or less than 1 w/v%.

Examples of the nutritional substance include meat extract, peptone, polypeptone, yeast extract, dry yeast, corn steep liquor, skim milk powder, hydrochloric acid hydrolysate of defatted soybean, extracts of animal or plant materials or microbial cells, and decomposition products thereof. A concentration of the nutritional substance in the medium is different depending on the nutritional substance used, but may be, for example, equal to or more than 0.1 w/v% and equal to or less than 10 w/v%.

Furthermore, vitamins can also be added as necessary. Examples of the vitamins include biotin, thiamine (vitamin B1), pyridoxine (vitamin B6), pantothenic acid, inositol, and nicotinic acid.

A pH of the medium is preferably equal to or more than 6 and equal to or less than 8.

Specific examples of a preferred medium include A medium [Inui, M. et al., Metabolic analysis of Corynebacterium glutamicum during lactate and succinate productions under oxygen deprivation conditions. J. Mol. Microbiol. Biotechnol. 7:182-196 (2004)] and BT medium [Oumumasa, C. A. et al., Corynebacterium glutamicum glycerldehyde-3-phosphate dehydrogenase isoforms with opposite, ATP-dependent regulation. J. Mol. Microbiol. Biotechnol. 8:91-103 (2004)]. In these media, a concentration of sugar may be within the above-described range.

### (Raw material compound)

The raw material compound is preferably a compound which can be taken into cells by the transformant, and more preferably a compound which is easily industrially utilized, such as a compound which is contained in a plant in a large amount.

Among the above-described raw material compounds, as sugars, a sugar which can generate glucose by metabolism of the transformant is preferable, and glucose is more preferable. Such sugars include an oligosaccharide and a polysaccharide, having a glucose unit. Examples of the above-described oligosaccharide and polysaccharide include monosaccharides such as fructose, mannose, arabinose, xylose, and galactose; disaccharides such as cellobiose, sucrose, lactose, maltose, trehalose, and xylobiose; and polysaccharides such as dextrin and soluble starch. In addition, it is preferable to use glucose from the viewpoint of improving manufacturing efficiency of the 4-hydroxybenzoic acid composition according to the second embodiment and the third embodiment.

In addition, examples of the compound from which the transformant can generate chorismic acid by metabolism, among the above-described raw material compounds, include quinic acid and shikimic acid.

In addition, for example, a raw material derived from a plant can be used as a raw material containing these raw material compounds. As the raw material derived from a plant, for example, molasses or a saccharified solution containing a plurality of sugars such as glucose, which is obtained by saccharifying, with a saccharifying enzyme, non-edible agricultural waste such as straw (rice straw, barley straw, wheat straw, rye straw, oat straw, and the like), bagasse, and corn stover, energy crops such as switchgrass, napier grass, and miscanthus, or wood chips and waste paper, can also be used.

Among these, as the raw material compound, glucose, chorismic acid, quinic acid, or shikimic acid is preferable, and as a raw material containing these compounds, a raw material derived from a plant is preferably used.

### (Reaction solution)

As the reaction solution, a natural reaction solution or a synthetic reaction solution, containing a carbon source, a nitrogen source, inorganic salts, and the like, can be used.

As the carbon source, the raw material compound described above or the molasses and the saccharified solution containing the raw material compound may be used. In addition to sugars, as the carbon source, sugar alcohols such as mannitol, sorbitol, xylitol, and glycerin, organic acids such as acetic acid, citric acid, lactic acid, fumaric acid, maleic acid, and gluconic acid, alcohols such as ethanol and propanol, hydrocarbons such as normal paraffin, and the like can also be used.

The carbon source can be used alone or in combination of two or more kinds thereof.

A concentration of the raw material compound in the reaction solution is preferably equal to or more than 1 w/v% and equal to or less than 20 w/v%, more preferably equal to or more than 2 w/v% and equal to or less than 10 w/v%, and still more preferably equal to or more than 2 w/v% and equal to or less than 5 w/v%.

In addition, a concentration of the total carbon source containing the raw material compound may be equal to or more than 2 w/v% and equal to or less than 5 w/v%.

As the nitrogen source, inorganic or organic ammonium compounds such as ammonium chloride, ammonium sulfate, ammonium nitrate, and ammonium acetate; urea, aqueous ammonia, sodium nitrate, potassium nitrate; and the like can be used. In addition, nitrogen-containing organic compounds such as corn steep liquor, meat extract, peptone, NZ-amine, protein hydrolysate, and amino acid can also be used. The nitrogen source can be used alone or in combination of two or more kinds thereof. A concentration of the nitrogen source in the reaction solution is different depending on the nitrogen compound used, but may be, for example, equal to or more than 0.1 w/v% and equal to or less than 10 w/v%.

Examples of the inorganic salts include potassium dihydrogen phosphate, potassium hydrogen phosphate, magnesium sulfate, sodium chloride, iron(II) sulfate, iron(III) nitrate, manganese sulfate, zinc sulfate, cobalt sulfate, and calcium carbonate. The inorganic salts can be used alone or in combination of two or more kinds thereof. A concentration of the inorganic salts in the reaction solution is different depending on the inorganic salts used, but may be, for example, equal to or more than 0.01 w/v% and equal to or less than 1 w/v%.

Examples of the nutritional substance include meat extract, peptone, polypeptone, yeast extract, dry yeast, corn steep liquor, skim milk powder, hydrochloric acid hydrolysate of defatted soybean, extracts of animal or plant materials or microbial cells, and decomposition products thereof. A concentration of the nutritional substance in the medium is different depending on the nutritional substance used, but may be, for example, equal to or more than 0.1 w/v% and equal to or less than 10 w/v%.

Furthermore, vitamins can also be added as necessary. Examples of the vitamins include biotin, thiamine (vitamin B1), pyridoxine (vitamin B6), pantothenic acid, inositol, and nicotinic acid.

A pH of the reaction solution is preferably equal to or more than 6 and equal to or less than 8.

Specific examples of a preferred reaction solution include the A medium and the BT medium described above. In these reaction solutions, a concentration of sugar may be within the above-described range.

### (Reaction conditions)

From the viewpoint of manufacturing efficiency of the 4-hydroxybenzoic acid composition, a reaction temperature, that is, a growth temperature of the transformant is preferably equal to or higher than 20°C, more preferably equal to or higher than 23°C, and still more preferably equal to or higher than 25°C. In addition, from the same viewpoint, the above-described growth temperature is preferably equal to or lower than 50°C, more preferably equal to or lower than 48°C, and still more preferably equal to or lower than 47°C.

From the viewpoint of manufacturing efficiency of the 4-hydroxybenzoic acid composition, a reaction time is preferably equal to or shorter than 7 days, more preferably equal to or shorter than 5 days, and still more preferably equal to or shorter than 3 days. In addition, the above-described reaction time is not particularly limited as long as the reaction sufficiently proceeds, but is, for example, equal to or longer than 1 day.

The culture may be any of a batch type, a fed-batch type, or a continuous type. Among these, from the viewpoint of manufacturing efficiency of the 4-hydroxybenzoic acid composition, a batch type is preferable.

The reaction may be performed under aerobic conditions or reducing conditions. Production ability of the 4-hydroxybenzoic acid composition by the transformant itself is higher under aerobic conditions. However, in the aerobic conditions, the transformant proliferates, so that the raw material compound is consumed for the proliferation, and thus the manufacturing efficiency of the 4-hydroxybenzoic acid composition is reduced.

Therefore, it is preferable to perform the reaction under aerobic conditions and conditions in which the transformant does not proliferate. In the present specification, "not proliferating" includes substantially not proliferating or hardly proliferating. For example, by using a reaction solution in which one or more of a compound essential for proliferation of a microorganism, such as vitamins including biotin, thiamine, and the like, and a nitrogen source, is deficient or limited, the proliferation of the transformant can be inhibited or suppressed.

In addition, in the reducing conditions, since the transformant does not substantially proliferate, the manufacturing efficiency of the 4-hydroxybenzoic acid composition is improved because the raw material compound is not consumed for the proliferation.

The reducing conditions are defined by a redox potential of the reaction solution. The redox potential of the reaction solution is preferably equal to or more than -500 mV and equal to or less than -200 mV, and more preferably equal to or more than -500 mV and equal to or less than -150 mV.

A reduction state of the reaction solution can be simply estimated by a resazurin indicator (bleaching from blue to colorless in the reduction state), but can be accurately measured using a redox potential difference meter (for example, ORP Electrodes manufactured by Broadley-James Corporation).

As a method for adjusting the reaction solution under the reducing conditions, any known method can be used without limitation. For example, as a liquid medium of the reaction solution, an aqueous solution for the reaction solution may be used instead of distilled water or the like, and a method for adjusting the aqueous solution for the reaction solution can be referred to, for example, a culture solution adjustment method for an absolute anaerobic microorganism such as a sulfate-reducing microorganism (Pfennig, N. et al., (1981): The dissimilatory sulfate-reducing bacteria, In The Prokaryotes, A Handbook on Habitats Isolation and Identification of Bacteria, Ed. by Starr, M. P. et al., p926-940, Berlin, Springer Verlag.) or "Agricultural Chemistry Experiment Book, Vol. 3, edited by the Department of Agricultural Chemistry, Faculty of Agriculture, Kyoto University, 26th edition, 1990, published by Sangyo Tosho Co., Ltd.", and an aqueous solution under the desired reducing conditions can be obtained.

Specifically, by heating or decompressing distilled water or the like to remove dissolved gas, the aqueous solution for the reaction solution under reducing conditions can be obtained. In this case, by treating the distilled water or the like under a pressure of preferably equal to or less than 10 mmHg, more preferably equal to or less than 5 mmHg, and still more preferably equal to or less than 3 mmHg, and under conditions of preferably equal to or longer than 1 minute and equal to or shorter than 60 minutes, and more preferably equal to or longer than 5 minutes and equal to or shorter than 40 minutes, the dissolved gas, particularly dissolved oxygen can be removed to prepare the aqueous solution for the reaction solution under reducing conditions.

In addition, the aqueous solution for the reaction solution under reducing conditions can be adjusted by adding an appropriate reducing agent (for example, thioglycolic acid, ascorbic acid, cysteine hydrochloride, mercaptoacetic acid, thiolacetic acid, glutathione, sodium sulfide, and the like).

It is also an effective method for adjusting the aqueous solution for the reaction solution under reducing conditions to appropriately combine these methods.

In a case where the reaction is performed under reducing conditions, it is preferable that the reaction solution is maintained under reducing conditions during the reaction. In order to maintain the reducing conditions during the reaction, it is desirable to prevent oxygen from the reaction system from being mixed as much as possible; and specific examples thereof include a method of sealing the reaction system with an inert gas such as nitrogen gas and carbon dioxide. As a method for more effectively preventing the oxygen from being mixed, in some cases, it is necessary to appropriately add a pH maintenance adjustment solution to the reaction system or various nutrient dissolution solutions in order to efficiently function the metabolic function in the transformant of the second embodiment and the third embodiment during the reaction; but in such a case, it is effective to remove oxygen from the addition solution in advance.

### [(B) Step of removing impurities in reaction solution]

Next, the step (B) of removing impurities in the reaction solution is performed. By culturing as described above, the 4-hydroxybenzoic acid composition is produced in the reaction solution. The 4-hydroxybenzoic acid composition can be recovered by recovery in the reaction solution, but the reaction solution contains components that affect the polymerizability and color tone of the 4-hydroxybenzoic acid composition, such as the transformant, the unreacted raw material compound, byproducts of the transformant, and components contained in the reaction solution itself. Therefore, by removing these components from the reaction solution, the polymerizability and color tone of the 4-hydroxybenzoic acid composition can be further improved.

Examples of the components that affect the polymerizability and color tone of the 4-hydroxybenzoic acid composition include various intermediate metabolites produced by the transformant, substances contained in the "other components" and "medium" described above, and specific elements.

Examples of the above-described intermediate metabolites include shikimic acid, dehydroshikimic acid, quinic acid, protocatechuic acid, and amino acids, but the intermediate metabolites are not limited thereto. In addition, examples of the above-described specific elements include nitrogen, phosphorus, potassium, magnesium, sodium, iron, manganese, zinc, and cobalt, but the specific elements are not limited thereto.

As a method of removing the impurities in the reaction solution in a case of producing the 4-hydroxybenzoic acid composition according to the second embodiment and the third embodiment, both isolating of the transformant and adsorption of the chemical component are performed. By performing both the separation of the transformant and the adsorption of the chemical component, the impurities can be further removed from the 4-hydroxybenzoic acid composition, and as a result, the polymerizability and color tone of the 4-hydroxybenzoic acid composition can be further improved.

First, the isolating method of the transformant will be described.

As the isolating method of the transformant, a known isolating method can be used, and examples thereof include collection of a supernatant liquid by standing, membrane filtration, and centrifugal separation. These methods can be appropriately combined and performed.

As a treatment condition by the membrane filtration, the treatment can be performed under general filtration conditions. From the viewpoint of improving the manufacturing efficiency of the 4-hydroxybenzoic acid composition, a membrane pore diameter is preferably equal to or more than 0.01 µm, more preferably equal to or more than 0.03 µm, still more preferably equal to or more than 0.05 µm, even more preferably equal to or more than 0.07 µm, even still more preferably equal to or more than 0.10 µm, further more preferably equal to or more than 0.15 µm, and even further more preferably equal to or more than 0.20 µm. In addition, from the same viewpoint, the membrane pore diameter is preferably equal to or less than 3 µm, more preferably equal to or less than 2 µm, and still more preferably equal to or less than 1 µm. Examples of a method of measuring the membrane pore diameter include a general measurement method using a mercury intrusion method, a bubble point test, a bacterial filtration method, and the like; but it is preferable to use a value obtained by the bubble point test. Examples of a material of the membrane used for the membrane filtration include a polymer membrane, a ceramic membrane, and a stainless steel membrane.

As a centrifuge used for the centrifugal separation, general equipment such as a isolating plate type, a cylindrical type, and a decanter type can be used. From the viewpoint of improving the manufacturing efficiency of the 4-hydroxybenzoic acid composition, a lower limit value of a temperature during the centrifugal separation is preferably equal to or higher than 5°C, more preferably equal to or higher than 10°C, and still more preferably equal to or higher than 20°C. In addition, from the same viewpoint, the upper limit value of the temperature during the centrifugal separation is preferably equal to or lower than 70°C, more preferably equal to or lower than 60°C, and still more preferably equal to or lower than 40°C.

In addition, a relative centrifugal force and a time during the centrifugal separation can be appropriately set, but from the viewpoint of improving the polymerizability and color tone of the 4-hydroxybenzoic acid composition, the lower limit value of the relative centrifugal force is preferably equal to or more than 3,000 g, more preferably equal to or more than 5,000 g, still more preferably equal to or more than 7,000 g, even more preferably equal to or more than 10,000 g, and even still more preferably equal to or more than 15,000 g. In addition, from the viewpoint of improving the manufacturing efficiency of the 4-hydroxybenzoic acid composition, the upper limit value of the relative centrifugal force is preferably equal to or less than 50,000 g, more preferably equal to or less than 25,000 g, and still more preferably equal to or less than 20,000 g.

Similarly, from the viewpoint of improving the polymerizability and color tone of the 4-hydroxybenzoic acid composition, the lower limit value of the time is preferably equal to or longer than 0.2 minutes, more preferably equal to or longer than 0.5 minutes, and still more preferably equal to or longer than 1 minute. In addition, from the viewpoint of improving the manufacturing efficiency of the 4-hydroxybenzoic acid composition, the upper limit value of the time is preferably equal to or shorter than 75 minutes, more preferably equal to or shorter than 60 minutes, and still more preferably equal to or shorter than 30 minutes.

Next, the adsorption method of the chemical component will be described.

Examples of the adsorption method of the chemical component include a method of adding an adsorbent having no activity against 4-hydroxybenzoic acid to the reaction solution after the transformant is removed by the above-described method.

The adsorbent is not particularly limited as long as it has no activity against 4-hydroxybenzoic acid and can adsorb the unreacted raw material compound and the components contained in the reaction solution itself. Examples of such an adsorbent include inorganic adsorbents such as a natural adsorbent (natural zeolite, silver zeolite, acid clay, and the like) and a synthetic adsorbent (synthetic zeolite, molecular sieve, bacteria-adsorbing polymer, hydroxyapatite, phosphate rock, titanium silicate, silica gel, silica-alumina gel, porous glass, and the like); activated carbon such as powdered activated carbon, granular activated carbon, fibrous activated carbon, block activated carbon, extruded activated carbon, molded activated carbon, synthetic granular activated carbon, and synthetic fiber activated carbon; and organic adsorbents such as a molecular adsorption resin, an ion exchange resin, an ion exchange fiber, a chelating resin, and a chelating fiber.

Among these, from the viewpoint of environmental compatibility, it is preferable to use activated carbon.

In the second embodiment, from the viewpoint of further improving the color tone of the 4-hydroxybenzoic acid composition, an amount of the adsorbent to be added is preferably equal to or more than 0.1 g, more preferably equal to or more than 0.2 g, still more preferably equal to or more than 0.3 g, and even more preferably equal to or more than 0.4 g, with respect to 100 g of the reaction solution.

In addition, in the second embodiment, the upper limit value of the amount of the adsorbent to be added is not particularly limited, but from the viewpoint of work efficiency of the step (C) of recovering the 4-hydroxybenzoic acid composition in the reaction solution, which will be described later, the upper limit value thereof is preferably equal to or less than 5.0 g, more preferably equal to or less than 4.0 g, still more preferably equal to or less than 3.0 g, even more preferably equal to or less than 2.0 g, even still more preferably equal to or less than 1.0 g, and further more preferably equal to or less than 0.5 g, with respect to 100 g of the reaction solution.

In the third embodiment, from the viewpoint of further improving the color tone of the 4-hydroxybenzoic acid composition, an amount of the adsorbent to be added is preferably equal to or more than 0.3 g, more preferably equal to or more than 0.5 g, still more preferably equal to or more than 0.8 g, even more preferably equal to or more than 1.0 g, even still more preferably equal to or more than 1.5 g, further more preferably equal to or more than 1.8 g, and even further more preferably equal to or more than 2.0 g, with respect to 100 g of the reaction solution.

In addition, in the third embodiment, the upper limit value of the amount of the adsorbent to be added is not particularly limited, but from the viewpoint of work efficiency of the step (C) of recovering the 4-hydroxybenzoic acid composition in the reaction solution, which will be described later, the upper limit value thereof is preferably equal to or less than 5.0 g, more preferably equal to or less than 4.0 g, still more preferably equal to or less than 3.0 g, even more preferably equal to or less than 2.5 g, and even still more preferably equal to or less than 2.1 g, with respect to 100 g of the reaction solution.

### [(C) Step of recovering 4-hydroxybenzoic acid composition in reaction solution]

Next, the step (C) of recovering the 4-hydroxybenzoic acid composition in the reaction solution is performed. By removing the impurities as described above, it is possible to obtain a reaction solution containing the 4-hydroxybenzoic acid composition having a relatively high purity, but the 4-hydroxybenzoic acid composition can be further isolated from the reaction solution by a known method. Examples of such a known method include a crystallization method, a membrane isolating method, an organic solvent extraction method, and various adsorption methods (using an ion exchange resin, a synthetic adsorbent, or the like).

In addition, after the recovery of the 4-hydroxybenzoic acid composition, a step of washing the 4-hydroxybenzoic acid composition or a step of purifying the 4-hydroxybenzoic acid composition may be provided as necessary.

As the step of washing the 4-hydroxybenzoic acid composition, a known washing method can be used. Examples of the known washing method include ultrasonic washing, stirring washing, circulation washing, and immersion washing. Among these, stirring washing is preferable.

As a washing solvent, a washing solvent which does not dissolve the 4-hydroxybenzoic acid composition but dissolves only the impurities is preferable. As such a washing solvent, for example, pure water is preferable. Here, the "pure water" refers to water having a conductivity of equal to or more than 1 MΩ·cm. The water having the above-described conductivity has a high purity, and the impurities contained therein, such as chlorine and salts, are extremely small.

From the viewpoint of further improving the polymerizability and color tone of the 4-hydroxybenzoic acid composition, an amount of the washing solvent in the step of washing the 4-hydroxybenzoic acid composition is preferably equal to or more than 7 times, more preferably equal to or more than 8 times, still more preferably equal to or more than 9 times, and even more preferably equal to or more than 10 times with respect to the mass of the obtained 4-hydroxybenzoic acid composition.

In addition, from the viewpoint of improving the manufacturing efficiency of the 4-hydroxybenzoic acid composition, the amount of the washing solvent is preferably equal to or less than 50 times, more preferably equal to or less than 25 times, still more preferably equal to or less than 15 times, and even more preferably equal to or less than 12 times.

From the viewpoint of further improving the polymerizability and color tone of the 4-hydroxybenzoic acid composition, the number of times of washing in the step of washing the 4-hydroxybenzoic acid composition is preferably equal to or more than 1, more preferably equal to or more than 2, and still more preferably equal to or more than 3.

In addition, from the viewpoint of improving the manufacturing efficiency of the 4-hydroxybenzoic acid composition, the number of times of washing is preferably equal to or less than 10, more preferably equal to or less than 7, still more preferably equal to or less than 5, and even more preferably equal to or less than 4.

### [Use]

The 4-hydroxybenzoic acid composition according to the second embodiment and the third embodiment can be used for the same applications as a 4-hydroxybenzoic acid composition synthesized by a petroleum chemical process in the related art, using a fossil resource as a raw material. That is, examples of the application of the 4-hydroxybenzoic acid composition according to the second embodiment and the third embodiment include a polymer raw material such as polyester, a polyester resin, a polyester fiber, a liquid crystal polyester, a liquid crystal polyester resin, and a liquid crystal polyester fiber; a synthesis raw material of paraben which is a preservative, a dye, a pigment, an adhesive resin, and a plasticizer.

Among these, it is preferable to use for a polymer application. That is, the polymer using the 4-hydroxybenzoic acid composition according to the second embodiment and the third embodiment is a polymer containing at least a structure of 4-hydroxybenzoic acid (A) derived from the 4-hydroxybenzoic acid composition according to the second embodiment or 4-hydroxybenzoic acid (A) derived from the 4-hydroxybenzoic acid composition according to the third embodiment.

As described above, the 4-hydroxybenzoic acid composition according to the second embodiment and the third embodiment is improved in the balance between the environmental compatibility and the polymerizability or the balance between the environmental compatibility and the color tone. Therefore, the 4-hydroxybenzoic acid composition according to the second embodiment and the third embodiment can be preferably used as a polymer (biobased polymer) for a biomass plastic in which the environmental compatibility and the polymerizability or the environmental compatibility and the color tone are highly compatible.

Examples of products made from the polymer using the 4-hydroxybenzoic acid composition according to the second embodiment and the third embodiment include various power cords, electrical equipment, various components, building materials, furniture, fishing lines and other fishing gear, fishing nets, ropes, and liquid crystal polymers.

As the polymer according to the second embodiment and the third embodiment, a liquid crystal polymer is preferable. The structure of 4-hydroxybenzoic acid (A) contained in the polymer according to the second embodiment and the structure of 4-hydroxybenzoic acid (A) contained in the polymer according to the third embodiment are a molecular shape which is elongated and flat, and have a molecular chain having high rigidity along a long chain of the molecule (this molecular chain having high rigidity is referred to as, for example, a "mesogen skeleton"). As described above, the 4-hydroxybenzoic acid composition according to the second embodiment and the third embodiment is improved in the balance between the environmental compatibility and the polymerizability or the balance between the environmental compatibility and the color tone. That is, the polymer according to the second embodiment and the third embodiment can be suitably used as a liquid crystal polymer in which the balance between the environmental compatibility, the thermal conductivity, and the mechanical strength is improved, which is derived from the improvement in the balance between the environmental compatibility and the polymerizability or the balance between the environmental compatibility and the color tone of the 4-hydroxybenzoic acid composition.

Here, as the liquid crystal polymer, an additive such as a reinforcing agent may be contained in the polymer according to the second embodiment and the third embodiment, depending on the necessary characteristics as long as the characteristics of the downstream product obtained using the liquid crystal polymer are not significantly impaired.

Examples of the additive include fibrous reinforcing agents such as glass fiber, silica alumina fiber, alumina fiber, and carbon fiber; acicular reinforcing agents such as aluminum borate whisker and potassium titanate whisker; inorganic fillers such as glass beads, talc, mica, graphite, wollastonite, and dolomite; release improving agents such as fluororesin and metal soaps; colorants such as a dye and a pigment; an antioxidant; a heat stabilizer; an ultraviolet absorber; an antistatic agent; and a surfactant. These additives may be used in combination of two or more kinds thereof.

In addition, an additive having an external lubricant effect, such as a higher fatty acid, a higher fatty acid ester, a higher fatty acid metal salt, and a fluorocarbon-based surfactant, can also be used. Furthermore, in a case of a small amount, a thermoplastic resin (for example, polyamide, crystalline polyester, polyphenylene sulfide, polyether ketone, polycarbonate, polyphenylene ether and a modified product thereof, polysulfone, polyethersulfone, polyetherimide, and the like) or a thermosetting resin (for example, a phenolic resin, an epoxy resin, and the like) other than the polymer according to the second embodiment and the third embodiment may be contained. In a case of using a thermoplastic resin or a thermosetting resin other than the polymer according to the second embodiment and the third embodiment, it is necessary to select the kind and the addition amount thereof such that liquid crystallinity and formability of the polymer itself according to the second embodiment and the third embodiment are not impaired.

In the product of the polymer using the 4-hydroxybenzoic acid composition according to the second embodiment and the third embodiment, a proportion of the 4-hydroxybenzoic acid composition according to the second embodiment and the third embodiment to the entire 4-hydroxybenzoic acid used as a raw material for a predetermined processed matter (product) may be defined for each manufacturer.

By defining the proportion of the 4-hydroxybenzoic acid composition according to the second embodiment and the third embodiment for each manufacturer, the actually measured biobased content of the product to be discharged or discarded is measured, and thus the manufacturer who is subject to the obligation to recover the product can be specified. In addition, in a certain manufacturer, the proportion of the 4-hydroxybenzoic acid composition according to the second embodiment and the third embodiment to the entire 4-hydroxybenzoic acid may be defined for each manufacturing lot of the product; and in this case, the manufacturing lot can be traced. In the traceability provision method in the related art (for example, mixing of a pigment or the like), there is a concern that the strength of various products may be reduced; but according to the 4-hydroxybenzoic acid composition according to the second embodiment and the third embodiment, the desired polymerizability and color tone can be obtained, so that the strength of various products can be secured while the manufacturer can be specified.

The embodiments of the present invention have been described above, but these are examples of the present invention and various configurations other than the above can be adopted. In addition, the present invention is not limited to the above-described embodiments, and modifications, improvements, and the like within the range in which the object of the present invention can be achieved are included in the present invention.

Specifically, in the second embodiment and the third embodiment, the 4-hydroxybenzoic acid has been described as an example of a chemical substance using a biomass resource as a raw material, but the technical idea of the present disclosure can be applied to various substances produced using a biomass resource as a raw material, including phenol and the like.

In addition, in the second embodiment, an embodiment in which the concentration of shikimic acid contained in 4-hydroxybenzoic acid has been defined has been described as an example; but the present disclosure also includes inventions in which the contents of various intermediate metabolites produced by the transformant, substances contained in the "other components", "medium", and the like described above, specific elements, and the like are defined.

Here, examples of the above-described intermediate metabolites include shikimic acid, dehydroshikimic acid, quinic acid, protocatechuic acid, and amino acids, but the intermediate metabolites are not limited thereto. In addition, examples of the above-described specific elements include nitrogen, phosphorus, potassium, magnesium, sodium, iron, manganese, zinc, and cobalt, but the specific elements are not limited thereto.

### [EXAMPLES]

Embodiments of the present invention will be described in detail based on Examples and Comparative Examples. As a reminder, the present invention is not limited to Examples. Here, Examples and Comparative Examples related to the first embodiment of the present invention are Examples 1a to 3a and Comparative Examples 1a to 3a; Examples and Comparative Examples related to the second embodiment of the present invention are Examples 1b to 3b and Comparative Examples 1b to 3b; and Examples and Comparative Examples related to the third embodiment of the present invention are Examples 1c to 3c and Comparative Examples 1c to 3c.

### <Examples and Comparative Examples>

### (Example 1a)

One platinum loop of a coryneform bacterial transformant HBA-47 (Accession No.: NITE BP-01849) described in JP6327654B was inoculated into a test tube containing 2% by mass of calcium carbonate in 10 ml of an A liquid medium containing kanamycin 50 µg/ml [obtained by dissolving 2 g of (NH₂)₂CO, 7 g of (NH₄)₂SO₄, 0.5 g of KH₂PO₄, 0.5 g of K₂HPO₄, 0.5 g of MgSO₄·7H₂O, 1 ml of 0.06% (w/v) FeSO₄·7H₂O + 0.042% (w/v) MnSO₄·2H₂O, 1 ml of 0.02% (w/v) biotin solution, 2 ml of 0.01% (w/v) thiamine solution, 2 g of yeast extract, 7 g of vitamin assay casamino acid, and 40 g of glucose in 1 L of distilled water], and the mixture was aerobically shaken and cultured at 33°C, 24 hours, and 200 rpm. Next, the above-described HBA-47 was isolated from the above-described test solution by vacuum filtration using a membrane filter (Steritop 0.22 µm × 33 mm, manufactured by Merck KGaA) having a pore diameter of 0.22 µm, thereby obtaining a test solution.

Thereafter, a coryneform bacterial transformant PHE21 (Accession No.: NITE BP-996) produced by a method described in Example 1a and paragraph 0135 of JP5932660B was added to the above-described test solution such that the final concentration of the bacterial cells was 10%, thereby obtaining a bacterial cell suspension. The bacterial cell suspension was placed in a 100 ml medium bottle, and subjected to a reaction by adding sodium-4-hydroxybenzoate as a substrate such that the concentration was 250 mM under reducing conditions (redox potential: -450 mV), while stirring in a water bath kept at 33°C. In this case, the reaction was carried out while controlling the pH of the reaction solution with a pH controller (model number: DT-1023, manufactured by Able Co., Ltd.) using 2.5 N ammonia water such that the pH of the reaction solution did not fall below 7.0. The reaction solution was centrifuged (4°C, 15,000 g, 10 minutes) to obtain, as a supernatant liquid, a solution containing a phenol composition synthesized from glucose (phenol concentration: 0.8% by mass).

The solution was continuously distilled to concentrate the phenol concentration to be 4.3% by mass, and then dehydrated by pervaporation (60°C, 50 Torr) using a DDR-type zeolite membrane to concentrate the phenol concentration to be 22% by mass. The concentrated solution was allowed to stand at room temperature to be two-phase isolated into a phenol phase and an aqueous phase, and the phenol phase was recovered (phenol concentration: 72% by mass). The phenol phase was further dehydrated using an evaporator (60°C/20 hPa) and concentrated to a concentration of 99% by mass. Furthermore, the residual moisture was vaporized by an evaporator (150°C/20 hPa) to obtain a phenol composition.

### (Example 2a)

A phenol composition was obtained by the same method as in Example 1a, except that the conditions of the pervaporation using the DDR-type zeolite membrane were set to 85°C and 250 Torr.

### (Example 3a)

The phenol composition obtained in Example 1a was stored in a transparent glass bottle at room temperature and in the air for half a year to obtain a phenol composition.

### (Comparative Example 1a)

A phenol composition was obtained by the same method as in Example 1a, except that the residual moisture was not vaporized by the evaporator (150°C·20 hPa).

### (Comparative Example 2a)

A commercially available phenol product (manufactured by FUJIFILM Wako Pure Chemical Corporation) was prepared.

### (Comparative Example 3a)

A waste liquid containing phenol (phenol concentration: 6.2% by mass) generated in a case of synthesizing a phenolic resin by the method described in "(Production of phenolic resin)" below using the phenol product of Comparative Example 2a was prepared. The solution was continuously distilled to concentrate the phenol concentration to be 7.0% by mass, and then dehydrated by pervaporation (60°C, 50 Torr) using a DDR-type zeolite membrane to concentrate the phenol concentration to be 29% by mass. The concentrated solution was allowed to stand at room temperature to be two-phase isolated into a phenol phase and an aqueous phase, and the phenol phase was recovered (phenol concentration: 72% by mass). Furthermore, the moisture was further removed by an evaporator (60°C·20 hPa), and the phenol concentration was concentrated to be 99% by mass to obtain a phenol composition.

### (Production of phenolic resin)

A phenolic resin was produced using the phenol compositions produced in Examples 1a to 3a and Comparative Examples 1a to 3a.

100 parts by mass of the phenol composition obtained in each of Examples 1a to 3a and Comparative Examples 1a to 3a, 2 parts by mass of oxalic acid dihydrate, and 82 parts by mass of 37% formalin aqueous solution were reacted with each other in a reactor equipped with a stirring device, a reflux condenser, and a thermometer at 100°C for 3 hours. Thereafter, the mixture was heated and raised to 180°C at a vacuum degree of 40 torr for 90 minutes, water was distilled and removed, the unreacted phenol composition was distilled and removed while blowing steam at 180°C for 30 minutes, and the distilled and removed water and phenol composition were used as a waste liquid. A phenolic resin using the phenol composition of each of Examples 1a to 3a and Comparative Examples 1a to 3a was obtained.

### (Evaluation of physical properties)

Each physical property of the phenol composition obtained in each of Examples 1a to 3a and Comparative Examples 1a to 3a was evaluated by the following method.

### (Phenol concentration analysis)

The quantification of the phenol in the phenol composition of each of Examples 1a to 3a and Comparative Examples 1a to 3a was performed by gas chromatography based on JIS K2437.

The phenol composition of each of Examples 1a to 3a and Comparative Examples 1a to 3a was dissolved in pure water to have a concentration of 90% by mass, thereby obtaining an analysis sample. The analysis sample was analyzed by gas chromatography in which a blank was obtained by dissolving the phenol composition (manufactured by FUJIFILM Wako Pure Chemical Corporation) at a concentration of 90% by mass in pure water.

Analysis conditions of the gas chromatography are shown in <Analysis conditions> below. The results are shown in Table 1.

### <Analysis conditions>

GC device: GC-2030 (manufactured by Shimadzu Corporation)
Carrier gas: N₂
Detector: hydrogen flame ionization (FID) detector; FID temperature: 300°C
· Column: SH-RXi-1HT, inner diameter: 0.25, length: 30 m, film thickness: 0.25 µm (manufactured by Shimadzu GLC Ltd.)
· Vaporization chamber temperature: 210°C
· Column flow rate: 0.64 mL/min
· Column temperature rising conditions: hold at 50°C for 5 min, temperature rise to 300°C at 20°C/min, and hold at 300°C for 10 min

### (Actually measured biobased content)

A content of radioactive carbon ¹⁴C in all carbon atoms in the phenol (A) of the phenol composition of each of Examples 1a to 3a and Comparative Examples 1a to 3a was measured based on biobased concentration test specification ASTM D6866-20 defined by American Society for Testing and Materials (ASTM). Next, from the measurement result and a concentration of radioactive carbon ¹⁴C in circulating carbon in the 1950s, a concentration of radioactive carbon ¹⁴C in all carbon atoms in a case where the concentration of radioactive carbon ¹⁴C in circulating carbon in the 1950s in the phenol (A) in the phenol composition of each of Examples 1a to 3a and Comparative Examples 1a to 3a was set to 100% was calculated as an actually measured biobased content, and evaluated as an indicator of environmental compatibility. The calculation results are shown in Table 1.

### (Absorbance)

The phenol composition of each of Examples 1a to 3a and Comparative Examples 1a to 3a was dissolved in pure water such that the phenol concentration was 90% by mass to prepare a measurement sample. For the measurement sample, an absorbance of the measurement sample at a wavelength of 425 nm when pure water was used as a blank was measured using an ultraviolet-visible spectrophotometer (UV-2400PC, manufactured by Shimadzu Corporation). The results are shown in Table 1.

### (Color tone)

The phenolic resin produced using the phenol composition of each of Examples 1a to 3a and Comparative Examples 1a to 3a was heated at 130°C for 10 minutes according to "(Production of phenolic resin)" described above to produce a molten sample. Thereafter, the color of the molten sample was quantified based on the iodine color number measured in accordance with DIN6162, and evaluated according to the following criteria. The evaluation results are shown in Table 1.
A (light yellow): iodine color number of equal to or more than 1 and less than 6
B (yellow): iodine color number of equal to or more than 6 and less than 9
C (yellowish brown): iodine color number of equal to or more than 9 and less than 44
D (dark brown): iodine color number of equal to or more than 44

**[Table 1]**

| | Phenol concentration [% by mass] | Actually measured biobased content [%] | Absorbance | Color tone |
|---|---|---|---|---|
| Example 1a | > 98 | > 95 | 0.077 | A |
| Example 2a | > 98 | > 95 | 0.224 | B |
| Example 3a | > 98 | > 95 | 0.620 | C |
| Comparative Example 1a | > 98 | > 95 | 0.959 | D |
| Comparative Example 2a | > 98 | 0 | 0.013 | A |
| Comparative Example 3a | > 98 | 0 | 0.746 | D |

As shown in Table 1, the balance between the environmental compatibility and the color tone of the obtained phenolic resin was improved by setting the absorbance of the phenol composition to be equal to or more than 0.001 and equal to or less than 0.70.

### (Example 1b)

One platinum loop of a coryneform bacterial transformant HBA-47 (Accession No.: NITE BP-01849) described in JP6327654B was inoculated into a test tube containing 2% by mass of calcium carbonate in 10 ml of an A liquid medium containing kanamycin 50 µg/ml [obtained by dissolving 2 g of (NH₂)₂CO, 7 g of (NH₄)₂SO₄, 0.5 g of KH₂PO₄, 0.5 g of K₂HPO₄, 0.5 g of MgSO₄·7H₂O, 1 ml of 0.06% (w/v) FeSO₄·7H₂O + 0.042% (w/v) MnSO₄·2H₂O, 1 ml of 0.02% (w/v) biotin solution, 2 ml of 0.01% (w/v) thiamine solution, 2 g of yeast extract, 7 g of vitamin assay casamino acid, and 40 g of glucose in 1 L of distilled water], and the mixture was aerobically shaken and cultured at 33°C, 24 hours, and 200 rpm. As a result, a test solution containing 4-hydroxybenzoic acid synthesized from glucose was obtained (4-hydroxybenzoic acid concentration: 7.3% by mass).

The above-described HBA-47 was isolated from the above-described test solution by vacuum filtration using a membrane filter (Steritop 0.22 µm × 33 mm, manufactured by Merck KGaA) having a pore diameter of 0.22 µm. Next, powdered activated carbon was added to the above-described test solution to carry out an activated carbon treatment. 0.4 g of the powdered activated carbon was used for 100 g of the test solution. Next, a crystallization operation of precipitating a solute was carried out by adding 25% sulfuric acid to the test solution subjected to the activated carbon treatment to lower the pH from 8 to 3. Next, the precipitated solute was filtered and isolated to recover a solid precipitate. Next, the obtained precipitate was stirred and washed three times with 10 times the mass of the precipitate in pure water at 200 rpm using a stirrer, and then dried to obtain a solid 4-hydroxybenzoic acid composition.

### (Example 2b)

A solid 4-hydroxybenzoic acid composition was obtained by the same method as in Example 1b, except that the number of times of stirring and washing the obtained precipitate in pure water with 10 times the mass of the precipitate was changed to 2.

### (Example 3b)

A solid 4-hydroxybenzoic acid composition was obtained by the same method as in Example 1b, except that the number of times of stirring and washing the obtained precipitate in pure water with 10 times the mass of the precipitate was changed to 1.

### (Comparative Example 1b)

A solid 4-hydroxybenzoic acid composition was obtained by the same method as in Example 1b, except that the washing method of the obtained precipitate was changed to a method of stirring and washing the precipitate once with 5 times the amount of pure water.

### (Comparative Example 2b)

A solid 4-hydroxybenzoic acid composition was obtained by the same method as in Example 1b, except that the washing method of the obtained precipitate was changed as follows.

The obtained precipitate was dissolved in 10 times the amount of methanol, and the insoluble fraction was removed by filtration. Thereafter, the pressure was gradually reduced to equal to or less than 100 hPa at 40°C, and methanol was vaporized until the liquid component of methanol disappeared to obtain a solid 4-hydroxybenzoic acid composition.

### (Comparative Example 3b)

A commercially available 4-hydroxybenzoic acid (manufactured by Tokyo Chemical Industry Co., Ltd., product code: H0207) was prepared.

### (Evaluation of physical properties)

Each physical property of the 4-hydroxybenzoic acid composition obtained in each of Examples 1b to 3b and Comparative Examples 1b to 3b was evaluated by the following method.

### (Concentration analysis)

The quantification of the concentration of 4-hydroxybenzoic acid and shikimic acid in the 4-hydroxybenzoic acid composition of each of Examples 1b to 3b and Comparative Examples 1b to 3b was carried out by high-performance liquid chromatography using a high-performance liquid chromatograph (Chromaster (registered trademark), manufactured by Hitachi High-Tech Science Corporation) equipped with a UV detector under the following <Analysis conditions>. The results are shown in Table 2.

### <Analysis conditions>

Column: COSMOSIL 5C18-AR-II (φ4.6 mm × 250 mm) manufactured by NACALAI TESQUE, INC.
Mobile phase: water/methanol/perchloric acid = 4/1/0.0075 (vol/vol/vol) isocratic elution
Flow rate: 1 mL/mmin
Column temperature: 40°C
Detection method: photodiode array (PDA) detector (210 nm)

### (Actually measured biobased content)

A content of radioactive carbon ¹⁴C in all carbon atoms in the 4-hydroxybenzoic acid (A) of the 4-hydroxybenzoic acid composition of each of Examples 1b to 3b and Comparative Examples 1b to 3b was measured based on biobased concentration test specification ASTM D6866-20 defined by American Society for Testing and Materials (ASTM). Next, from the measurement result and a concentration of radioactive carbon ¹⁴C in circulating carbon in the 1950s, a concentration of radioactive carbon ¹⁴C in all carbon atoms in a case where the concentration of radioactive carbon ¹⁴C in circulating carbon in the 1950s in the 4-hydroxybenzoic acid (A) in the 4-hydroxybenzoic acid composition of each of Examples 1b to 3b and Comparative Examples 1b to 3b was set to 100% was calculated as an actually measured biobased content, and evaluated as an indicator of environmental compatibility. The calculation results are shown in Table 2.

### (Polymerizability)

241.7 g (4-hydroxybenzoic acid content: 1.75 mol) of the 4-hydroxybenzoic acid composition of each of Examples 1b to 3b and Comparative Examples 1b to 3b, 93.1 g (0.5 mol) of biphenyl (manufactured by Tokyo Chemical Industry Co., Ltd., product code: B0464), 83.0 g (0.5 mol) of terephthalic acid (manufactured by Tokyo Chemical Industry Co., Ltd., product code: T0166), and 326.4 g (3.2 mol) of acetic anhydride (manufactured by Tokyo Chemical Industry Co., Ltd., product code: A2036) were charged into a 1 L polymerization vessel equipped with a distillation device, a stirrer, and a nitrogen introduction pipe. After carrying out acetoxylation reaction at 150°C for 3 hours, the temperature was raised to 240°C, continued for 6 hours, and then further raised to 300°C, and the polymerization was continued until the contents were solidified. The polymerized substance (prepolymer) taken out was pulverized and then solid-phase polymerized in nitrogen at 300°C, and the polycondensation was completed at a point in time at which a predetermined stirring torque was reached, thereby obtaining a liquid crystal polymer.

The obtained liquid crystal polymer was injection-molded under conditions of a resin temperature of 340°C, a mold temperature of 150°C, and an injection pressure of 110 MPa to produce a bending test piece having a length of 80 mm, a width of 10 mm, and a thickness of 4 mm. Using the produced bending test piece, a bending strength was measured in accordance with ASTM D790.

The polymerizability was evaluated as follows from the obtained bending strength. The results are shown in Table 2.
A: bending strength of equal to or more than 100 MPa
B: bending strength of equal to or more than 90 MPa and less than 100 MPa
C: bending strength of less than 90 Mpa

**[Table 2]**

| | 4-Hydroxybenzoic acid concentration [wt%] | Actually measured biobased content [%] | Shikimic acid concentration [wt%] | Polymerizability |
|---|---|---|---|---|
| Example 1b | > 99 | > 95 | 0.000 | A |
| Example 2b | > 99 | > 95 | 0.005 | A |
| Example 3b | > 99 | > 95 | 0.010 | B |
| Comparative Example 1b | > 99 | > 95 | 0.013 | C |
| Comparative Example 2b | > 99 | > 95 | 0.020 | C |
| Comparative Example 3b | > 99 | 0 | 0.000 | A |

As shown in Table 2, the balance between the environmental compatibility and the polymerizability was improved by setting the content of shikimic acid contained in the 4-hydroxybenzoic acid composition to be equal to or less than a predetermined concentration.

### (Example 1c)

One platinum loop of a coryneform bacterial transformant HBA-47 (Accession No.: NITE BP-01849) described in JP6327654B was inoculated into a test tube containing 2% by mass of calcium carbonate in 10 ml of an A liquid medium containing kanamycin 50 µg/ml [obtained by dissolving 2 g of (NH₂)₂CO, 7 g of (NH₄)₂SO₄, 0.5 g of KH₂PO₄, 0.5 g of K₂HPO₄, 0.5 g of MgSO₄·7H₂O, 1 ml of 0.06% (w/v) FeSO₄·7H₂O + 0.042% (w/v) MnSO₄·2H₂O, 1 ml of 0.02% (w/v) biotin solution, 2 ml of 0.01% (w/v) thiamine solution, 2 g of yeast extract, 7 g of vitamin assay casamino acid, and 40 g of glucose in 1 L of distilled water], and the mixture was aerobically shaken and cultured at 33°C, 24 hours, and 200 rpm. As a result, a test solution containing 4-hydroxybenzoic acid synthesized from glucose was obtained (4-hydroxybenzoic acid concentration: 7.3% by mass).

The above-described HBA-47 was isolated from the above-described test solution by vacuum filtration using a membrane filter (Steritop 0.22 µm × 33 mm, manufactured by Merck KGaA) having a pore diameter of 0.22 µm. Next, powdered activated carbon was added to the above-described test solution to carry out an activated carbon treatment. 2.0 g of the powdered activated carbon was used for 100 g of the test solution. Next, a crystallization operation of precipitating a solute was carried out by adding 25% sulfuric acid to the test solution subjected to the activated carbon treatment to lower the pH from 8 to 3. Next, the precipitated solute was filtered and isolated to recover a solid precipitate. Next, the obtained precipitate was stirred and washed three times with 10 times the mass of the precipitate in pure water at 200 rpm using a stirrer, and then dried to obtain a solid 4-hydroxybenzoic acid composition.

### (Example 2c)

A solid 4-hydroxybenzoic acid composition was obtained by the same method as in Example 1c, except that the addition amount of the powdered activated carbon to the test solution was changed to 1.0 g.

### (Example 3c)

A solid 4-hydroxybenzoic acid composition was obtained by the same method as in Example 1c, except that the addition amount of the powdered activated carbon to the test solution was changed to 0.4 g.

### (Comparative Example 1c)

A solid 4-hydroxybenzoic acid composition was obtained by the same method as in Example 1c, except that the addition amount of the powdered activated carbon to the test solution was changed to 0.2 g.

### (Comparative Example 2c)

A solid 4-hydroxybenzoic acid composition was obtained by the same method as in Example 1c, except that the addition amount of the powdered activated carbon to the test solution was changed to 0.4 g, and the number of times of stirring and washing the obtained precipitate in pure water with 10 times the mass of the precipitate was changed to 2.

### (Comparative Example 3c)

A commercially available 4-hydroxybenzoic acid (manufactured by Tokyo Chemical Industry Co., Ltd., product code: H0207) was prepared.

### (Evaluation of physical properties)

Each physical property of the 4-hydroxybenzoic acid composition obtained in each of Examples 1c to 3c and Comparative Examples 1c to 3c was evaluated by the following method.

### (Concentration analysis)

The quantification of the concentration of 4-hydroxybenzoic acid in the 4-hydroxybenzoic acid composition of each of Examples 1c to 3c and Comparative Examples 1c to 3c was carried out by high-performance liquid chromatography using the above-described high-performance liquid chromatograph (Chromaster (registered trademark), manufactured by Hitachi High-Tech Science Corporation) equipped with a UV detector under the following <Analysis conditions>. The results are shown in Table 3.

### <Analysis conditions>

Column: COSMOSIL 5C18-AR-II (φ4.6 mm × 250 mm) manufactured by NACALAI TESQUE, INC.
Mobile phase: water/methanol/perchloric acid = 4/1/0.0075 (vol/vol/vol) isocratic elution
Flow rate: 1 mL/mmin
Column temperature: 40°C
Detection method: photodiode array (PDA) detector (210 nm)

### (Actually measured biobased content)

A content of radioactive carbon ¹⁴C in all carbon atoms in the 4-hydroxybenzoic acid (A) of the 4-hydroxybenzoic acid composition of each of Examples 1c to 3c and Comparative Examples 1c to 3c was measured based on biobased concentration test specification ASTM D6866-20 defined by American Society for Testing and Materials (ASTM). Next, from the measurement result and a concentration of radioactive carbon ¹⁴C in circulating carbon in the 1950s, a concentration of radioactive carbon ¹⁴C in all carbon atoms in a case where the concentration of radioactive carbon ¹⁴C in circulating carbon in the 1950s in the 4-hydroxybenzoic acid (A) in the 4-hydroxybenzoic acid composition of each of Examples 1c to 3c and Comparative Examples 1c to 3c was set to 100% was calculated as an actually measured biobased content, and evaluated as an indicator of environmental compatibility. The calculation results are shown in Table 3.

### (Absorbance)

The 4-hydroxybenzoic acid composition of each of Examples 1c to 3c and Comparative Examples 1c to 3c was dissolved in ethanol to have a concentration of 20 g/L to prepare a measurement sample. For the measurement sample, an absorbance of the measurement sample at a wavelength of 425 nm when pure water was used as a blank was measured using an ultraviolet-visible spectrophotometer (UV-2400PC, manufactured by Shimadzu Corporation). The results are shown in Table 3.

### (Color tone)

241.7 g (4-hydroxybenzoic acid content: 1.75 mol) of the 4-hydroxybenzoic acid composition of each of Examples 1c to 3c and Comparative Examples 1c to 3c was charged into a 1 L reactor equipped with a stirrer and a nitrogen introduction pipe. After introducing 1 L of nitrogen into the reactor, the temperature was raised to 240°C, and the mixture was heated in a nitrogen atmosphere for 6 hours. After the heating, the 4-hydroxybenzoic acid composition in the reactor was dissolved in ethanol to have a concentration of 20% by mass, and the color of the solution was quantified based on the iodine color number measured in accordance with DIN6162, and evaluated according to the following criteria. The evaluation results are shown in Table 3.
A: iodine color number of equal to or more than 1 and equal to or less than 10
B: iodine color number of more than 10 and equal to or less than 20
C: iodine color number of more than 20

**[Table 3]**

| | 4-Hydroxybenzoic acid concentration [wt%] | Actually measured biobased content [%] | Absorbance | Color tone |
|---|---|---|---|---|
| Example 1c | > 99 | > 95 | 0.005 | A |
| Example 2c | > 99 | > 95 | 0.008 | A |
| Example 3c | > 99 | > 95 | 0.014 | B |
| Comparative Example 1c | > 99 | > 95 | 0.018 | C |
| Comparative Example 2c | > 99 | > 95 | 0.016 | C |
| Comparative Example 3c | > 99 | 0 | < 0.001 | A |

As shown in Table 3, the balance between the environmental compatibility and the color tone was improved by setting the absorbance of the 4-hydroxybenzoic acid composition to be equal to or less than 0.015.

Corynebacterium glutamicum PHE7 was deposited with NITE Patent Microorganisms Depositary (NPMD), National Institute of Technology and Evaluation (NITE), 2-5-8 Kazusakamatari, Kisarazu-shi, Chiba 292-0818, Japan (domestic deposit accepted on August 31, 2010; international deposit under the Budapest Treaty accepted on August 12, 2011; Accession No.: NITE BP-976).

Corynebacterium glutamicum PHE18 was deposited with NITE Patent Microorganisms Depositary (NPMD), National Institute of Technology and Evaluation (NITE), 2-5-8 Kazusakamatari, Kisarazu-shi, Chiba 292-0818, Japan (domestic deposit accepted on October 21, 2010; international deposit under the Budapest Treaty accepted on September 28, 2011; Accession No.: NITE BP-995).

Corynebacterium glutamicum PHE21 was deposited with NITE Patent Microorganisms Depositary (NPMD), National Institute of Technology and Evaluation (NITE), 2-5-8 Kazusakamatari, Kisarazu-shi, Chiba 292-0818, Japan (domestic deposit accepted on October 21, 2010; international deposit under the Budapest Treaty accepted on September 28, 2011; Accession No.: NITE BP-996) .

Corynebacterium glutamicum PHE31 was deposited with NITE Patent Microorganisms Depositary (NPMD), National Institute of Technology and Evaluation (NITE), 2-5-8 Kazusakamatari, Kisarazu-shi, Chiba 292-0818, Japan (domestic deposit accepted on November 2, 2010; international deposit under the Budapest Treaty accepted on September 28, 2011; Accession No.: NITE BP-999).

Corynebacterium glutamicum HBA-2 was deposited with NITE Patent Microorganisms Depositary (NPMD), National Institute of Technology and Evaluation (NITE), 2-5-8 Kazusakamatari, Kisarazu-shi, Chiba 292-0818, Japan (domestic deposit accepted on March 27, 2014; international deposit under the Budapest Treaty accepted on February 23, 2015; Accession No.: NITE BP-01838).

Corynebacterium glutamicum HBA-47 was deposited with NITE Patent Microorganisms Depositary (NPMD), National Institute of Technology and Evaluation (NITE), 2-5-8 Kazusakamatari, Kisarazu-shi, Chiba 292-0818, Japan (international deposit under the Budapest Treaty accepted on April 25, 2014; Accession No.: NITE BP-01849).

These strains have been internationally deposited under the Budapest Treaty, and are available to the public under the conditions set forth in 37 C.F.R. §1.808.

Priority is claimed on Japanese Patent Application No. 2023-096317, Japanese Patent Application No. 2023-096338, and Japanese Patent Application No. 2023-096350, filed on June 12, 2023, the disclosure of which is incorporated herein by reference.

| | | |
|---|---|---|
| 0-1 | Form PCT/RO/134 | |
| | The indications concerning the deposited microorganisms or other biological materials | |
| 0-1-1 | (PCT Rule 13bis) have been prepared on the basis of the information given on the right. | **JP0-PAS i520** |
| 0-2 | International application number | |
| 0-3 | Applicant's or agent's file reference | **SB-3039W001** |
| | | |
| 1 | The following indications relate to microorganisms or biological materials described in the detailed description of the invention. | |
| 1-1 | Paragraph number | 0199 |
| 1-3 | Indications of deposit | |
| 1-3-1 | Name of depository institution | NPMD, Independent Administrative Institution National Institute of Technology and Evaluation (NITE), Patent Microorganisms Depositary Center (NPMD) |
| 1-3-2 | Address of depository institution | Japan, 122, 2-5-8 Kazusa-Kamatari, Kisarazu-shi, Chiba 292-0818 |
| 1-3-3 | Date of deposit | 12 August 2011 (12.08.2011) |
| 1-3-4 | Accession number | **NPMD NITE BP-976** |
| 1-5 | Designated States for the purposes of this indication | **All designated States** |
| 2 | The following indications relate to microorganisms or biological materials described in the detailed description of the invention. | |
| 2-1 | Paragraph number | **0200** |
| 2-3 | Indications of deposit | |
| 2-3-1 | Name of depository institution | NPMD, Independent Administrative Institution National Institute of Technology and Evaluation (NITE), Patent Microorganisms Depositary Center (NPMD) |
| 2-3-2 | Address of depository institution | Japan, 122, 2-5-8 Kazusa-Kamatari, Kisarazu-shi, Chiba 292-0818 |
| 2-3-3 | Date of deposit | 28 September 2011 (28.09.2011) |
| 2-3-4 | Accession number | **NPMD NITE BP-995** |
| 2-5 | Designated States for the purposes of this indication | All designated States |
| 3 | The following indications relate to microorganisms or biologial materials described in the detailed description of the **invention.** | |
| 3-1 | **Paragraph number** | **0201** |
| 3-3 | Indications of deposit | |
| 3-3-1 | Name of depository institution | NPMD, Independent Administrative Institution National Institute of Technology and Evaluation (NITE), Patent Microorganisms Depositary Center (NPMD) |
| 3-3-2 | Address of depository institution | Japan, 122, 2-5-8 Kazusa-Kamatari, Kisarazu-shi, Chiba 292-0818 |
| 3-3-3 | Date of deposit | 28 September 2011 (28.09.2011) |
| 3-3-4 | Accession number | **NPMD NITE BP-996** |
| 3-5 | Designated States for the purposes of this indication | All designated States |
| 4 | The following indications relate to microorganisms or biological materials deacribed in the detailed description of the inwnticn. | |
| 4-1 | Paragraph number | **0202** |
| 4-3 | Indications of deposit | |
| 4-3-1 | Name of depository institution | NPMD, Independent Administrative Institution National Institute of Technology and Evaluation (NITE), Patent Microorganisms Depositary Center (NPMD) |
| 4-3-2 | Address of depository institution | Japan, 122, 2-5-8 Kazusa-Kamatari, Kisarazu-shi, Chiba 292-0818 |
| 4-3-3 | Date of deposit | 28 September 2011 (28.09.2011) |
| 4-3-4 | Accession number | **NPMD NITE BP-999** |
| 4-5 | Designated States for the purposes of this indication | All designated States |
| 5 | The following indications relate to microorganisms or biological materials described in the detailed description of the irvertion. | |
| 5-1 | Paragraph number | **0203** |
| 5-3 | Indications of deposit | |
| 5-3-1 | Name of depository institution | NPMD, Independent Administrative Institution National Institute of Technology and Evaluation (NITE), Patent Microorganisms Depositary Center (NPMD) |
| 5-3-2 | Address of depository institution | Japan, 122, 2-5-8 Kazusa-Kamatari, Kisarazu-shi, Chiba 292-0818; |
| 5-3-3 | Date of deposit | 23 February 2015 (23.02.2015) |
| 5-3-4 | Accession number | **NPMD NITE BP-01838** |
| 5-5 | Designated States for the purposes of this indication | All designated States |
| 6 | The following indications relate to microorganisms or biological materials described in the detailed description of the invention. | |
| 6-1 | Paragraph number | **0204** |
| 6-3 | Indications of deposit | |
| 6-3-1 | Name of depository institution | NPMD, Independent Administrative Institution National Institute of Technology and Evaluation (NITE), Patent Microorganisms Depositary Center (NPMD) |
| 6-3-2 | Address of depository institution | Japan, 122, 2-5-8 Kazusa-Kamatari, Kisarazu-shi, Chiba 292-0818 |
| 6-3-3 | Date of deposit | 25 April 2014 (25.04.2014) |
| 6-3-4 | Accession number | **NPMD NITE BP-01849** |
| 6-5 | Designated States for the purposes of this indication | All designated States |

| For receiving Office use only | | |
|---|---|---|
| 0-4 | This sheet was received together with the international application (yes / no). | ✔ |
| 0-4-1 | Authorized officer | Nagai Tsuneo |

## Claims

1. An aromatic compound composition comprising, at least:
an aromatic compound (A) in which an actually measured biobased content representing a proportion of radioactive carbon ¹⁴C derived from a biomass resource, the actually measured biobased content being determined based on a concentration of radioactive carbon in circulating carbon in the 1950s as measured in accordance with ASTM D6866-20, is equal to or more than 80% and equal to or less than 100%,
wherein the aromatic compound (A) is phenol (A) or 4-hydroxybenzoic acid (A),
in a case where the aromatic compound (A) is the phenol (A), an absorbance at a wavelength of 425 nm when the aromatic compound composition is dissolved in pure water to have a concentration of 90% by mass is equal to or more than 0.001 and equal to or less than 0.70, and
in a case where the aromatic compound (A) is the 4-hydroxybenzoic acid (A), an absorbance at a wavelength of 425 nm when the aromatic compound composition is dissolved in ethanol at a concentration of 20 g/L is equal to or less than 0.015, or a content of shikimic acid in the aromatic compound composition is equal to or less than 0.012% by mass with respect to 100% by mass of the entire aromatic compound composition.

2. The aromatic compound composition according to Claim 1,
wherein the aromatic compound (A) is the phenol (A).

3. The aromatic compound composition according to Claim 2,
wherein a content of the phenol (A) is equal to or more than 95% by mass and equal to or less than 100% by mass.

4. The aromatic compound composition according to Claim 2 or 3,
wherein the actually measured biobased content of the phenol (A) is equal to or more than 95% and equal to or less than 100%.

5. The aromatic compound composition according to any one of Claims 2 to 4,
wherein the absorbance is equal to or less than 0.62.

6. The aromatic compound composition according to any one of Claims 2 to 5,
wherein the phenol (A) includes at least a structure derived from a benzene ring of a product of one or two or more transformants selected from a transformant HBA-2 (Accession No.: NITE BP-01838) and a transformant HBA-47 (Accession No.: NITE BP-01849).

7. The aromatic compound composition according to any one of Claims 2 to 6,
wherein the phenol (A) includes at least a product of one or two or more transformants selected from a transformant PHE7 (Accession No.: NITE BP-976), a transformant PHE18 (Accession No.: NITE BP-995), a transformant PHE21 (Accession No.: NITE BP-996), and a transformant PHE31 (Accession No.: NITE BP-999).

8. A polymer comprising, at least:
a structural unit derived from the phenol (A) in the aromatic compound composition according to any one of Claims 2 to 7.

9. The polymer according to Claim 8,
wherein the polymer is a phenolic resin.

10. The aromatic compound composition according to Claim 1,
wherein the aromatic compound (A) is the 4-hydroxybenzoic acid (A), and
the content of shikimic acid in the aromatic compound composition is equal to or less than 0.012% by mass with respect to 100% by mass of the entire aromatic compound composition.

11. The aromatic compound composition according to Claim 10,
wherein the content of shikimic acid is equal to or less than 0.008% by mass with respect to 100% by mass of the entire aromatic compound composition.

12. The aromatic compound composition according to Claim 1,
wherein the aromatic compound (A) is the 4-hydroxybenzoic acid (A), and
the absorbance at a wavelength of 425 nm when the aromatic compound composition is dissolved in ethanol at a concentration of 20 g/L is equal to or less than 0.015.

13. The aromatic compound composition according to Claim 12,
wherein the absorbance is equal to or less than 0.010.

14. The aromatic compound composition according to Claim 12 or 13,
wherein the absorbance is equal to or more than 0.001.

15. The aromatic compound composition according to any one of Claims 10 to 14,
wherein a content of the 4-hydroxybenzoic acid (A) is equal to or more than 95% by mass and equal to or less than 100% by mass with respect to 100% by mass of the entire aromatic compound composition.

16. The aromatic compound composition according to any one of Claims 10 to 15,
wherein the actually measured biobased content of the 4-hydroxybenzoic acid (A) is equal to or more than 95% and equal to or less than 100%.

17. The aromatic compound composition according to any one of Claims 10 to 16,
wherein the 4-hydroxybenzoic acid (A) includes at least a product of one or two transformants selected from a transformant HBA-2 (Accession No.: NITE BP-01838) and a transformant HBA-47 (Accession No.: NITE BP-01849).

18. A polymer comprising, at least:
a structure of the 4-hydroxybenzoic acid (A) derived from the aromatic compound composition according to any one of Claims 10 to 17.

19. The polymer according to Claim 18,
wherein the polymer is a liquid crystal polymer.
